(19) Europäisches Patentamt
European Patent Office
Office européen des brevets

(11) Publication number : **0 605 963 A2**

# (12) EUROPEAN PATENT APPLICATION

(21) Application number : **93309825.3**

(22) Date of filing : **07.12.93**

(51) Int. Cl.$^5$ : **C08G 65/32,** A61K 47/48, C12N 11/06

(30) Priority : **09.12.92 US 987739**
**07.04.93 US 45052**
**23.11.93 US 157343**

(43) Date of publication of application :
**13.07.94 Bulletin 94/28**

(84) Designated Contracting States :
**AT BE CH DE DK ES FR GB GR IE IT LI LU MC NL PT SE**

(71) Applicant : **ORTHO PHARMACEUTICAL CORPORATION**
**U.S. Route 202,**
**P.O. Box 300**
**Raritan, New Jersey 08869-0606 (US)**

(72) Inventor : **Wright, David E.**
**10827 Ashlar Place**
**San Diego, California 92131 (US)**

(74) Representative : **Fisher, Adrian John et al**
**CARPMAELS & RANSFORD**
**43 Bloomsbury Square**
**London WC1A 2RA (GB)**

(54) **Peg hydrazone and peg oxime linkage forming reagents and protein derivatives thereof.**

(57) The present invention provides methods and compounds for modifying polypeptides with PEG or other water-soluble organic polymers. Novel water-soluble polymer reagents are provided for.

The water-soluble polymer reagents of the subject invention include hydrazine, hydrazine carboxylate, semicarbazide, thiosemicarbazide, carbonic acid dihydrazide, carbazide, thiocarbazide, and arylhydrazide derivatives as well as oxylamine derivatives of water-soluble organic polymers, such as polyethylene glycol, polypropylene glycol, polyoxyethylated polyol, heparin, heparin fragments, dextran, polysaccharides, polyamino acids, and polyvinyl alcohol.

Also provided for, are polypeptides of interest derivatized by the subject water-soluble polymer reagents.

Kits for modifying polypeptides with the subject water-soluble polymer reagents are also provided.

EP 0 605 963 A2

## Field of the Invention

This invention relates to water-soluble polymers, such as monomethoxypoly(ethylene glycol), that are modified to form a hydrazone linkage with an aldehyde group on a protein, and the invention also relates to protein molecules modified by these water-soluble polymers.

This invention further relates to such water-soluble polymers that are modified to form an oxime linkage, and protein molecules modified thereby.

## Background Art

Protein and other similar organic molecules may be chemically modified by covalent conjugation to water-soluble organic polymers such as polyethylene glycol (PEG) The production of such protein conjugates is of interest because of the desirable properties conferred on polypeptides by the attachment of the water-soluble polymers. These desirable properties include increased solubility in aqueous solutions, increased stability during storage, reduced immunogenicity, increased resistance to enzymatic degradation, compatibility with a wider variety of drug administration systems, and increased in vivo half-life. These properties that are brought about by the derivatization of polypeptides with PEG or other water-soluble polymers are especially of interest when the polypeptide is to be used as a therapeutic agent injected into the body or when the polypeptide is to be used in assays, usually immunoassays, for the detection and/or quantification of a compound of interest.

The attachment of reporter groups, ligands, etc. to proteins through a glycoprotein's carbohydrate moiety has been described [Weber, P. and Hof, L. (1975) Biochem. Biophys. Res. Commun. 65, 1298-1302; O'Shannessy, D.J., Dobersen, M.J., and Quarles, R.H. (1984) Immunol. Lett. 8, 273-277; O'Shannessy, D.J. and Quarles, R.H. (1985) J. Appl. Biochem. 7, 347-355; Chua, M.-M., Fan, S.-T., and Karush, F. (1984) Biochim. Biophys. Acta 800, 291-300; O'Shannessy, D.J. and Wilchek, M. (1990) Anal. Biochem. 191, 1-8; Koppel, G.A. (1990) Bioconjugate Chem. 1, 13-23]. A number of groups have been covalently attached in this manner including biotin, fluorescent probes, anticancer compounds, and solid supports.

U.S. Patent No. 4,847,325 describes the possibility of synthesizing a PEG-amine, PEG-hydrazide or PEG-hydrazine and attaching it to a glycoprotein. However, no experimental evidence was given that these PEG-derivatives had been synthesized, that these PEG-derivatives could modify an oxidized glycoprotein, and what the resulting biological properties of these putative PEG-proteins might be.

The publication by Kogan, T.P., Synthetic Communications, 22(16), 2417-2424 (1992), describes the synthesis of a monomethoxypoly(ethylene glycol)hydrazide.

The enzyme peroxidase has been modified with PEG through its carbohydrate groups [Urrutigoity, M. and Souppe, J. (1989) Biocatalysis 2, 145-149]. In this modification PEG-diamine was reacted with oxidized peroxidase, and the resulting imine was reduced with borohydride to form a stable bond between PEG and the carbohydrate group on the protein. Three molecules of PEG-20,000 were attached to the enzyme. A possible problem in using PEG-diamine in this manner is intermolecular cross-linking taking place between the protein molecules with PEG diamine functioning as the cross-linker. Another drawback of using PEG-diamine is the consumption of two available aldehyde groups for each PEG-diamine attached to the protein, thus lowering the potential number of sites for PEG incorporation.

Besides the hydrazone forming mPEG derivatives, also synthesized is a series of oxime forming mPEG derivatives. Oximes are formed by the reaction of hydroxylamine or oxylamine derivatives with aldehyde or ketone groups. Polystyrene substituted benzophenone oximes have been used as supports for solid-phase peptide synthesis [DeGrado, W.F., and Kaiser, E.T. (1980) J. Org. Chem. 45, 1295-1300]. In this example the growing peptide chain is coupled to the oxime group via an ester linkage. The substituted oxime bond is quite stable, and even unsubstituted aldoximes show good stability towards the Beckmann rearrangement requiring 60 hr at 100° in the presence of silica gel to yield the reaction [March, J. (1985) Advanced Organic Chemistry, New York-John Wiley & Sons, pp 987-989]. The oxime linkage has been used to couple morpholinodoxorubicin to an antibody [Mueller, B.M., Wrasidlo, W.A., and Reisfeld, R.A. (1990) Bioconjugate Chem. 1, 325-330]. In this example the ketone group of morpholinodoxorubicin was reacted with aminooxyacetic acid. The newly coupled free acid group was activated, and morpholinodoxorubicin was linked to the free amino groups of lysine on a monoclonal antibody.

A number of proteins have been modified by PEG. For a review see Inada, Y., Yoshimoto, T. Matsushima, A., and Saito, Y. (1986) Trends Biotechnol. 4: 68-73. A number of patents have issued and applications published in this field as listed below: U.S. Pat. No. 4,179,337; U.S. Pat. No. 4,609,546; U.S. Pat. No. 4,261,973; U.S. Pat. No. 4,055,635; U.S. Pat. No. 3,960,830; U.S. Pat. No. 4,415,665; U.S. Pat. No. 4,412,989; U.S. Pat. No. 4,002,531; U.S. Pat. No. 4,414,147; U.S. Pat. No. 3,788,948; U.S. Pat. No. 4,732,863; U.S. Pat. No. 4,745,180; EP No. 152,847; EP No. 98,110 published January 11, 1984. The above patents and patent publications also describe the use of other water-soluble polymer protein modifying reagents including but not restricted to polypropylene glycol (PPG), polyoxyethylated polyol (POP), heparin, heparin fragments, dextran, polysaccharides, polyamino acids including proline, polyvinyl alcohol (PVA) and other water-soluble organic polymers.

A recent patent publication (WO90/12874) describes the preparation of an mPEG-EPO in which the EPO contains a cysteine residue introduced by genetic engineering. A cysteine specific mPEG-reagent is then covalently attached to the genetically engineered free sulfhydryl group. Only one mPEG molecule could be incorporated into EPO and no evidence of this incorporation was presented. Also no biological or biophysical properties of the resulting mPEG-EPO were described.

Erythropoietin is a glycoprotein which regulates red blood cell production. Erythropoietin exerts its biological effect by binding to receptors on erythroid precursors (Krantz, S.B., Blood 77: 419-434 (1991)). The binding of erythropoietin to its receptor causes erythroid precursors to proliferate and differentiate into mature red blood cells. Other growth factors such as interleukin 3 or granulocyte-macrophage colony-stimulating factor also are involved in erythropoiesis along with cofactors such as iron, folic acid, and vitamin B12. Currently erythropoietin is approved for use in anemia of chronic renal failure in both dialysis and predialysis patients and for the anemia of HIV infection and in combination with zidovudine therapy. Current uses for erythropoietin under study include anemia of cancer, presurgical autologous blood donation, and perisurgical adjuvant therapy.

Erythropoietin consists of 165 amino acids which includes two disulfide bridges. Erythropoietin has four carbohydrate chains emanating from the protein backbone. Three of the carbohydrate groups are N-linked and are attached to asparagines 24, 38, and 83. Also there is one O-linked carbohydrate group secured to serine 126. The carbohydrate chains are branched and consist of fucose, galactose, N-acetylgalactosamine, N-acetylglucosamine, mannose, and sialic acid. The carbohydrate composition of erythropoietin is heterogeneous as determined by Sasaki, H., Bothner, B., Dell, A., and Fukuda, M. (1987) J. Biol. Chem. 262, 12059-12076. Carbohydrate groups account for about 40% of the protein's weight. The carbohydrate groups on erythropoietin are believed to increase the solubility of erythropoietin and prolong its serum half-life.

Several limitations exist with respect to which polypeptides may be covalently conjugated to water-soluble polymers and the extent to which the polypeptides can be modified. Different water-soluble polymer reagents vary with respect to the functional groups that provide for coupling to amino acid residues in polypeptides of interest. Specific functional groups provide for the coupling of water-soluble polymers to specific amino acid residues.

Modification of lysine residues using different mPEG-reagents having different properties such as succinimidyl carbonate-PEG, succinimidyl succinate-PEG, imidate-PEG, cyanuric chloride-PEG, carbonyldiimidazole-PEG, and PEG-phenylcarbonate derivatives (4-nitrophenol and 2,4,5-trichlorophenol) have been described. Each reagent has its own specific property. The subject application involves new carbohydrate PEG modifying agents with different specificities to oxidized carbohydrate groups analogous to the different lysine modifying PEG-derivatives.

Glycoproteins, i.e., polypeptides covalently joined to a carbohydrate molecule or molecules, provide additional opportunities for providing different methods of water-soluble polymer derivatization of a polypeptide because of the presence of the carbohydrate moieties on the polypeptide. Water-soluble polymer reagents may be coupled directly to the carbohydrate moieties of glycoproteins as opposed to the amino acid polypeptide backbone, i.e., various functional groups present on the polypeptide, of the glycoprotein. It may be advantageous to couple water-soluble reagents to the carbohydrate moiety of a glycoprotein rather than to the polypeptide backbone amino acids because of differences in charge displacement, steric hinderance, amino acid residues at active sites, and other problems that may disrupt the structure and function of the polypeptide component of the water-soluble polymer modified glycoprotein.

By providing for water-soluble polymer reagents that may be coupled to the carbohydrate moiety of glycoproteins it may be possible to covalently conjugate water-soluble polymers to proteins without substantially adversely affecting the biological activity of proteins that would be adversely affected through coupling at other amino acid residues.

## SUMMARY OF THE INVENTION

The present invention provides methods and compositions for modifying polypeptides with derivatives of water-soluble organic polymers, i.e., water-soluble polymer reagents, that form a hydrazone linkage with an aldehyde group or group with similar chemical reactivity, e.g., ketones, lactols, activated carboxylic acids or activated carboxylic acid derivatives on a polypeptide. Novel hydrazide, semicarbazide, aryl hydrazide, thiosemicarbazide, hydrazide carboxylate, carbonic acid dihydrazide, carbazide, and thiocarbazide derivatives of

polyethylene glycol (PEG) and other water-soluble polymers are provided. One or more of the water-soluble polymer reagents may be coupled to individual polypeptides or similar organic molecules to form hydrazones that link the polypeptide to water-soluble polymers.

Another aspect of the subject invention is to provide for proteins, particularly glycoproteins, modified by the covalent attachment of hydrazone linkage water-soluble polymer derivatives.

Also disclosed are methods and compositions for modifying polypeptides with derivatives of water-soluble organic polymers that form an oxime linkage with the above-mentioned aldehyde or similarly reactive groups. Novel oxylamine derivatives as listed hereinbelow, of polyethylene glycol (PEG) and other water-soluble polymers are provided and wherein one or more of the water-soluble polymer reagents may be coupled to individual polypeptides or similar organic molecules to form oximes that link the polypeptide to water-soluble polymers.

Another aspect of the subject invention is to provide for proteins, particularly glycoproteins, modified by the covalent attachment of oxylamine water-soluble polymer derivatives.

The water-soluble polymer reagents of the subject invention include hydrazone linkage and oxime linkage forming derivatives of polyethylene glycol homopolymers, polypropylene glycol homopolymers, copolymers of ethylene glycol with propylene glycol, wherein said homopolymers and copolymers are unsubstituted or substituted at one end with an alkyl group, polyoxyethylated polyols, polyvinyl alcohol, polysaccharides, polyvinyl ethyl ethers, and $\alpha,\beta$-Poly[(2-hydroxyethyl)-DL-aspartamide] and other water-soluble organic polymers. Polyethylene glycol water-soluble polymers include polyethylene glycol where one of the terminal hydroxyl group is modified with an R group, i.e., RO-PEG, where R may be alkyl, aryl, alkyaryl, aroyl, alkanoyl, benzoyl, arylalkylethers, cycloalkyl, cycloalkylaryl, and the like. The water-soluble polymers listed are only exemplary of water-soluble polymers represented by P. Various derivatives of the specifically recited water-soluble polymers are also contemplated, provided that the derivatives are water-soluble. More preferably, the water-soluble polymer P is selected from the group consisting of polyethylene glycol and derivatives thereof, the monomethyl ether of polyethylene glycol (mPEG) being particularly preferred (so as to avoid cross-linking between proteins).

Polypeptides of interest for water-soluble polymer derivatization by the subject water-soluble polymer include hormones, lymphokines, cytokines, growth factors, enzymes, vaccine antigens, and antibodies. Water-soluble polymer derivatization of erythropoietin (EPO), especially recombinant erythropoietin, and precursors, intermediates and mimetics thereof, are of particular interest.

Another aspect of the invention is to provide erythropoietin that has been partially oxidized and subsequently combined with (i) a semicarbazide derivative of the monomethoxypoly (ethylene glycol) (mPEG), so as to produce mPEG derivatized erythropoietin molecules containing 17-25 mPEG molecules/molecule of erythropoietin (joined through hydrazone linkages), (ii) a carboxylate hydrazide derivative of mPEG so as to produce derivatized EPO containing about 22-32 mPEGs/EPO (joined through hydrazone linkages), and (iii) oxylamine derivatives of mPEG so as to produce derivatized EPO containing about 3-36 mPEGs/EPO (joined through oxime linkages), all as measured by gel filtration retention time.

Another aspect of the invention is to provide methods of activating polypeptides for covalent conjugation with the subject water-soluble polymer reagents.

## DESCRIPTION OF THE DRAWINGS

Figure 1a shows an HPLC chromatogram of EPO. Figure 1b shows an HPLC chromatogram of EPO modified with a hydrazine derivative of mPEG5000. Figure 1c shows an HPLC chromatogram of EPO modified with a succinimide ester of mPEG5000.

Figure 2 is a graph of the hematocrit level of mice treated with mPEG5000-EPO containing different amounts of attached mPEG (28, 18 and 12 mPEGs/molecule of EPO). The EPO derivatized with 18 or 28 mPEG5000/molecule are derivatized using the subject semicarbazide compound. EPO derivatized with 12 mPEG5000/molecule is derivatized using the subject hydrazide compound.

Figure 3 is a graph showing the ability of EPO, hydrazide mPEG5000 EPO (12 PEG/EPO), hydrazide mPEG12000-EPO (6 PEG/EPO), thiosemicarbazide mPEG5000-EPO (25 PEG/EPO), semicarbazide mPEG12000-EPO (14 PEG/EPO), and semicarbazide mPEG12000-EPO (29 PEG/EPO) to bind a monoclonal antibody specific for EPO in an ELISA assay.

Figure 4 is a graph showing a comparison of the biological activity of EPO when modified with either mPEG-Hydrazide (HY) or mPEG-Semicarbazide (SC). Two different molecular weights (8500 and 5000) of mPEG were used in the comparison. Mouse albumin is used as the control.

Figure 5 is a plot showing the hematocrit level of mice treated with mPEG8500-EPO containing different amounts of attached mPEG (34, 20 and 12 mPEGs). EPO derivatized with 34 or 20 mPEG85000/molecule are derivatized using the subject semicarbazide compound. EPO derivatized with 12 mPEG85000/molecule

is derivatized using the subject hydrazide compound. Mouse albumin is used as the control.

Figure 6 is a graph showing the results of ELISA assays for mPEG modified EPO using a Clinigen® EPO EIA test kit. In the legend, SC5-24 refers to semicarbazide mPEG5000 modified EPO with 24 molecules of mPEG/molecule of EPO, SC5-18 refers to the semicarbazide mPEG5000 modified EPO with 18 molecules of mPEG semicarbazide /molecule of EPO.

Figure 7 is a graph showing the circulating half-life of EPO in plasma. In the legend SC5-18-iv refers to the semicarbazide mPEG5000 modified EPO with 18 molecules of mPEG /molecule of EPO and injected intravenously, EPO-iv refers to injected intravenously.

Figure 8 is a graph showing changes in hematocrit level in response to injection with EPO. In the legend, the term SC5-18 refers to the semicarbazide mPEG5000 modified EPO with 18 molecules of mPEG /molecule of EPO, the term SC5-22 refers to the semicarbazide mPEG5000 modified EPO with 22 molecules of mPEG5000 /molecule of EPO, the term HY5-8 refers to the hydrazide mPEG5000 modified EPO with 8 molecules of mPEG5000 /molecule of EPO.

Figure 9 is a graph showing changes in hematocrit level in response to injection with EPO. In the legend, the term SC5-24 refers to the semicarbazide mPEG5000 modified EPO with 24 molecules of mPEG /molecule of EPO, the term TS5-25 refers to the thiosemicarbazide mPEG5000 modified EPO with 25 molecules of mPEG5000 /molecule of EPO, the term DH5-22 refers to the dihydrazide mPEG5000 modified EPO with 22 molecules of mPEG5000 /molecule of EPO, M.A. refers to the mouse albumin control.

Figure 10 is a graph showing changes in hematocrit level in response to injection with EPO. In the legend, the term TS5-17 refers to the thiosemicarbazide mPEG5000 modified EPO with 17 molecules of mPEG5000 /molecule of EPO, the term SC8.5-12 refers to the semihydrazide mPEG8500 modified EPO modified with 12 molecules of mPEG8500 /molecule of EPO, SC2-15 refers to the semicarbazide mPEG2000 modified EPO with 15 molecules of mPEG2000 /molecule of EPO, M.A. refers to the mouse albumin control.

Figure 11 is a graph showing changes in hematocrit level in response to injection with EPO. The legend is as follows: the term SC5-18 refers to the mPEG5000 semicarbazide modified EPO with 18 molecules of mPEG5000 /molecule of EPO, the term SC12-14 refers to the semicarbazide mPEG12,000 modified EPO with 14 molecules of mPEG12,000 /molecule of EPO, the term SC5-28 refers to the semihydrazide mPEG5000 modified EPO with 28 molecules of mPEG5000 /molecule of EPO, the term HY12-6 refers to the hydrazide mPEG12,000 modified EPO with 6 molecules of mPEG12,000 /molecule of EPO, M.A. refers to the mouse albumin control.

Figure 12 is a graph showing changes in hematocrit level in response to injection with EPO. In the legend, the term SC8.5-34 refers to the semicarbazide mPEG8500 modified EPO with 34 molecules of mPEG8500 /molecule of EPO, the term SC12-29 refers to the semicarbazide mPEG12,000 modified EPO with 29 molecules of mPEG12,000 /molecule of EPO, M.A. refers to the mouse albumin control.

Figure 13 is a graph of hematocrit levels in mice injected subcutaneously or intravenously EPO and EPO derivatives. The legend is as follows: EPO-SC refers to EPO injected subcutaneously, EPO-iv refers to EPO injected intravenously, SC5-28-SC refers to semicarbazide mPEG5000 modified EPO with 28 molecules of mPEG5000 /molecule of EPO injected subcutaneously, SC5-28-iv refers to semicarbazide mPEG5000 modified EPO with 28 molecules of mPEG5000 /molecule of EPO injected intravenously, HY12-6-SC refers to hydrazide mPEG12,000 modified EPO with 6 molecules of mPEG12,000 /molecule of EPO injected subcutaneously, HY12-6-iv refers to hydrazide mPEG12,000 modified EPO with 6 molecules of mPEG12,000 /molecule of EPO injected intravenously, M.A.-sc refers to mouse albumin control injected subcutaneously, M.A.-iv refers to mouse albumin control injected intravenously.

Figure 14 is a graph showing hematocrit levels in mice injected with multiple versus single doses of .1 micrograms of EPO. The legend is as follows: EPOx3sc refers to EPO injected subcutaneously three times a week, EPOx3iv refers to EPO injected intravenously three times a week, SC5-22x1sc refers to semicarbazide mPEG5000 modified EPO with 22 molecules of mPEG5000 /molecule of EPO injected subcutaneously once a week, SG5-22x1iv refers to semicarbazide mPEG5000 modified EPO with 22 molecules of mPEG5000 /molecule of EPO injected intravenously once a week, M.A.x3 refers to control mouse albumin injected intravenously three times a week.

Figure 15 is a graph showing hematocrit levels in mice with tumor necrosis factor $\alpha$(TNF$\alpha$)-induced anemia and injected with EPO and derivatives of EPO. The legend is as follows: TNF(5) refers to TNF injected over five days, T+EPO(5) refers to TNF and EPO injected simultaneously over a period of five days, T+EPO(2) refers to TNF injected over a period of five days and EPO injected on days 1 and 4, T+SC5-18(5) refers to TNF injected over a period of five days simultaneously with semicarbazide mPEG5000 modified EPO with 18 molecules of mPEG5000 /molecule of EPO, T+SC5-18(2) refers to TNF injected over a period of five days simultaneously with semicarbazide mPEG5000 modified EPO with 18 molecules of mPEG5000 /molecule of EPO injected on days 1 and 4, M-A refers to the mouse albumin control.

Figure 16 is a graph showing hematocrit changes in response to injection with EPO. In the legend, 18PEG-A refers to EPO modified with mPEG-0-$CH_2CH_2$-NH-CO-$ONH_2$, (formula XXI of the invention), with 18 mPEG molecules per molecule of EPO; 31 PEG-C refers to EPO modified with mPEG-0-$CH_2CH_2$-NH-CO-$CH_2$-$ONH_2$ (formula XXIV of the invention), with 31 mPEG/EPO; 25PEG-C refers to EPO modified with mPEG-O-$CH_2$-$CH_2$-NH-CO-$CH_2$-$ONH_2$ (formula XXIV of the invention), with 25 molecules of mPEG/molecule EPO.

Figure 17 is a graph showing hematocrit changes in response to injection with EPO. In the legend, 22 PEG-B refers to EPO modified with the oxime-derivatized mPEG-0-$CH_2CH_2$-$ONH_2$ (formula XXIII of the invention), with 22 mPEG molecules/molecule of EPO; 17 PEG-B refers to EPO modified with the same oxime linker having 17 mPEG molecules/molecule of EPO, and 12 PEG-B refers to EPO modified with the same oxime linker having 12 mPEG molecules/molecule of EPO.

Figure 18 is a graph showing the ability of EPO, m-PEG-O-$CH_2CH_2$-NH-CO-$ONH_2$ ("A") mPEG5000 EPO (18 PEG/EPO), mPEG-0-$CH_2CH_2$-$ONH_2$ ("B") mPEG5000 EPO (22 PEG/EPO), mPEG-0-$CH_2CH_2$-$ONH_2$("B") mPEG5000 EPO (17 PEG/EPO) ,mPEG-O-$CH_2CH_2$-$ONH_2$ ("B") mPEG5000 EPO (12 PEG/EPO), mPEG-O-$CH_2CH_2$-NH-CO-$CH_2$-$ONH_2$ ("C") mPEG5000 EPO (31 PEG/EPO), and mPEG-O-$CH_2CH_2$-NH-CO-$CH_2$-$ONH_2$ ("C") mPEG5000 EPO (25 PEG/EPO) to bind a monoclonal antibody specific for EPO in an ELISA assay.

Figure 19 is a graph showing EPO dependent cell proliferation using mPEG-EPOs. In the legend 18 PEG-A refers to EPO modified with mPEG-0-$CH_2CH_2$-NH-CO-0$NH_2$ (formula XXI) at 18 PEG/EPO, 22 PEG-B refers to EPO modified with mPEG-0-$CH_2CH_2$-$ONH_2$ (formula XXIII) at 22 PEG/EPO, 17 PEG-B refers to formula XXIII at 17 PEG/EPO, 12 PEG-B refers to formula XXIII at 12 PEG/EPO, 31 PEG-C refers to EPO modified with mPEG-O-$CH_2CH_2$-NH-CO-$CH_2$-$ONH_2$ (formula XXIV) at 31 PEG/EPO, and 25 PEG-C refers to formula XXIV at 25 PEG/EPO.

Figure 20 is a graph showing hematocrit changes in response to injection with EPO. In the legend, 31 mPEGs refers to EPO modified with the oxime-derivatized mPEG-O-CO-$NHNH_2$ (formula II of the invention), having 31 mPEG molecules/molecule EPO.

## DESCRIPTION OF THE PREFERRED EMBODIMENTS

### Definitions

The term "water-soluble polymer reagent" as used herein, refers to a water-soluble polymer modified so as to contain a functional group that provides for the covalent conjugation of the water-soluble polymer to a polypeptide.

The term "polypeptide" as used herein, refers to polypeptides of various sizes, including larger polypeptides (frequently referred to as proteins), small peptides, and glycoproteins.

The term "oxidation activatable group" as used herein, refers to functional groups such as alcohols, polyols, lactols, amines, phenols, carboxylic acids, or carboxylic acid derivatives that react with the hydrazide portion or the oxylamine portion of the subject compounds after the functional group has been exposed to oxidative conditions. Oxidation activatable groups present on a polypeptide that is a glycoprotein may be present on the carbohydrate portion of the glycoprotein or on the amino acid residue portion of the glycoprotein. Exemplary, but not exclusive, of oxidation activatable groups are hydroxyl groups present on the carbohydrate portion of glycoproteins. The hydroxyl groups may be oxidized to hydrazide reactive aldehydes or oxylamine reactive aldehydes, depending on the derivative employed.

The term "partial oxidation" as used herein, refers to the processes of oxidation that proceed to an extent that does not completely abolish the biological activity of the polypeptide being oxidized.

The term "activated for conjugation" as used herein with respect to polypeptides, refers to the partial oxidation of a polypeptide, where the extent of oxidation is sufficient to convert at least one oxidation activatable group to a functional group capable of chemically reacting with the hydrazide portion or oxylamine portion (or similar functional group portion) of one of the subject water-soluble polymer reagents.

The term "biological activity" as used herein, refers to biologically relevant properties of a compound including: enzymatic activity, the ability to bind to receptors (including antibodies), the ability to bind ligands, the ability to induce an immune response, therapeutic activity and the like.

The term "antibodies," as used herein, includes both polyclonal and monoclonal antibodies with natural immunoglobulin sequences, synthetic antibody derivatives, and the like; antibodies may be modified so as to be joined to any of a variety of labels, fluorescent, radioactive, enzymatic, biotin/avidin or the like. Synthetic antibody derivatives include natural immunoglobulin sequences that have been mutated and selected for altered binding specificity, various immunoglobulin gene derived polypeptides, typically single chain, produced by genetically modified bacteria, antibodies modified so as to contain modified constant regions and the like; a review of such synthetic antibody derivatives based on the principles of antibody formation is provided in

Winter and Milstein, Nature, 349: 293-299 (1991).

The Invention

The subject invention provides novel polypeptide modifying reagents that are hydrazine or oxylamine derivatives of water-soluble polymers such as PEG, i.e, polyethylene glycol, for use in modifying polypeptides so as to be bound to water-soluble polymers. The water-soluble polymer reagents of the subject invention may be used to covalently attach a variety of water-soluble polymers to polypeptides of interest. The subject hydrazine and oxylamine derivatives of water-soluble polymers, i.e., water-soluble polymer reagents, may be covalently attached to proteins through reactions with aldehyde groups or other suitable functional groups present on the protein of interest. Aldehyde groups may be introduced by partially oxidizing the hydroxyl groups (or other oxidation activatable groups) on the polypeptide. Examples of oxidation activable groups include the hydroxyl groups present on the carbohydrate moieties of a glycoprotein. Suitable methods of oxidation, i.e., partial oxidation, include treating the polypeptide of interest with an oxidizing agent such as periodate or other oxidation agents known to those of skill in the art, or adding an enzyme capable of catalyzing oxidation reactions on portions of the protein of interest, e.g., galactose oxidase. Another aspect of the subject invention is to provide polypeptides modified by the reagent molecules, i.e., the subject water-soluble polymer hydrazine or oxylamine derivatives, so as to be covalently bonded to one or more water-soluble polypeptides.

Preferred formulae of the compounds useful for coupling water-soluble polymers to polypeptides are as follows:

HYDRAZINE DERIVATIVES

(I) $P-O-CH_2-CO-NHNH_2$,

a hydrazine derivative;

(II) $P-O-CO-NHNH_2$,

a hydrazine carboxylate derivative;

(III) $P-NH-CO-NHNH_2$,

a semicarbazide derivative;

(IV) $P-NH-CS-NHNH_2$,

a thiosemicarbazide derivative;

(V) $P-NHCO-NHNHCO-NHNH_2$,

a carbonic acid dihydrazide derivative;

(VI) $P-NHNHCONHNH_2$,

a carbazide derivative;

(VII) $P-NHNHCSNHNH_2$,

a thiocarbazide derivative;

(VIII) $P-NH-CO-C_6H_4-NHNH_2$,

an aryl hydrazide derivative;

(IX) $P-O-CO-CH_2CH_2-CO-NHNH_2$,

a hydrazide derivative;

OXYLAMINE DERIVATIVES

(XIX) $P-O-CH_2CH_2-CO-ONH_2$;

(XX) $P-O-CH_2CH_2-O-CO-ONH_2$;

(XXI) $P-O-CH_2CH_2-NH-CO-ONH_2$;

(XXII) $P-O-CH_2CH_2-NH-CS-ONH_2$;

(XXIII) $P-O-CH_2CH_2-ONH_2$;

(XXIV) $P-O-CH_2CH_2-NH-CO-CH_2ONH_2$;

(XXV) $P-O-CH_2CH_2-O-CO-CH_2-ONH_2$;

(XXVI) $P-O-CH_2CH_2-CH(OH)-CH_2-ONH_2$; and

(XXVII) $P-O-CH_2CH_2-CO-CH_2-ONH_2$.

P represents a water-soluble organic polymer in the above formulae. Water-soluble organic polymers of interest have hydroxyl groups appended to the polymer backbone and may be selected from known water-soluble polymers including but not limited to: (a) dextran and dextran derivatives, including dextran sulfate, P-amino cross linked dextrin, and carboxymethyl dextrin (b) cellulose and cellulose derivatives, including methylcellulose and carboxymethyl cellulose (c) starch and dextrines, and derivatives and hydroylactes of starch (d)

polyalklyene glycol and derivatives thereof, including polyethylene glycol, methoxypolyethylene glycol, polyethylene glycol homopolymers, polypropylene glycol homopolymers, copolymers of ethylene glycol with propylene glycol, wherein said homopolymers and copolymers are unsubstituted or substituted at one end with an alkyl group (e) heparin and fragments of heparin, (f) polyvinyl alcohol and polyvinyl ethyl ethers, (g) polyvinylpyrrolidone, (h) α,β-Poly[(2-hydroxyethyl)-DL-aspartamide, and (i) polyoxyethylated polyols. Preferably, the water-soluble polymer P is selected from dextran and dextran derivatives, dextrine and dextrine derivatives, and more preferably polyethylene glycol and derivatives thereof. Polyethylene glycol water-soluble polymers include polyethylene glycol where one of the terminal hydroxyl group is modified with an R group, i.e., RO-PEG, where R may be alkyl, aryl, alkyaryl, aroyl, alkanoyl, benzoyl, arylalkylethers, cycloalkyl, cycloalkylaryl, and the like. The water-soluble polymers listed are only exemplary of water-soluble polymers represented by P. Various derivatives of the specifically recited water-soluble polymers are also contemplated, provided that the derivatives are water-soluble. More preferably, the water-soluble polymer P is selected from the group consisting of polyethylene glycol and derivatives thereof, the monomethyl ether of polyethylene glycol (mPEG) being particularly preferred (so as to avoid cross-linking between proteins). When polypeptides modified by the water-soluble polymer reagents of the subject invention are to be used as pharmaceuticals, polymer P should be non-toxic.

The compounds of formulae I-IX may be represented generally by the formula:

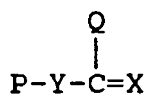

wherein X is O or S; Q is selected from the group consisting of $-NHNH_2$, and $-C_6H_4-NHNH_2$; and Y is selected from the group consisting of -O-, $-OCH_2-$, -NH-, -NHNH-, $-O-CO-CH_2CH_2-$ and -NHCO-N-NHNH-; and P is a water- soluble organic polymer (as in compounds I-IX).

The compounds of formulae XIX-XXVII may be represented generally by the formula:

P-Y-X-Q

wherein X is C=O, C=S, $CH_2$ or CHOH; Q is selected from the group consisting of $-ONH_2-$, and $-CH_2-ONH_2-$, and Y is selected from the group consisting of $-O-CH_2CH_2-$, $-O-CH_2CH_2-O-$, $-O-CH_2CH_2-N-$, $O-CH_2CH_2-S$, and $-O-CH_2CH_2CH-$; and P is a water soluble organic polymer (as in compounds XIX-XXVII).

In addition to the molecules of formulae I, II, III, IV, V, VI, VII, VIII, and IX the subject invention also includes polypeptides modified by reaction with the molecules of formulae I, II, III, IV, V, VI, VII, VIII, and IX. Polypeptides modified by the water-soluble polymer reagents of formulae I, II, III, IV, VI, VII, VIII, and IX may be represented by formulae X, XI, XII, XIII, XIV, XV, XVI, XVII, and XVIII, respectively:

HYDRAZIDE-MODIFIED POLYPEPTIDES

(X) $[P-O-CH_2-CO-NHN=CH-]_n-Z$,
(XI) $[P-O-CO-NHN=CH-]_n-Z$,
(XII) $[P-NH-CO-NHN=CH-]_n-Z$,
(XIII) $[P-NH-CS-NHN=CH-]_n-Z$,
(XIV) $[P-NHCO-N-NHNHCO-NHN=CH-]_n-Z$,
(XV) $[P-NHNCON=CH-]_n-Z$,
(XVI) $[P-NHNCSN=CH-]_n-Z$,
(XVII) $[P-NH-CO-C_6H_4-NHN=CH-]_n-Z$, and
(XVIII) $[P-O-CO-CH_2CH_2-CO-NHN=CH-]_n-Z$,

wherein P is a water-soluble polymer as previously described, Z represents a polypeptide, as described above, and n represents a number in the range 1 to x, where x is the maximum number of oxidation activatable groups present in polypeptide Z. The C in the hydrazone linkage formed between the water-soluble polymer reagent and Z was originally present on Z, not the water-soluble polymer reagent.

In addition to the molecules of formulae XIX, XX, XXI, XXII, XXIII, XXIV, XXV, XXVI and XXVII the subject invention also includes polypeptides modified by reaction with the molecules of formulae XIX, XX, XXI, XXII, XXIII, XXIV, XXV, XXVI and XXVII. Polypeptides modified by the water-soluble polymer reagents of formulae XIX, XX, XXI, XXII, XXIII, XXIV, XXV, XXVI and XXVII may be represented by formulae XXVIII, XXIX, XXX, XXXI, XXXII, XXXIII, XXXIV, XXXV and XXXVI, respectively:

OXYLAMINE-MODIFIED POLYPEPTIDES

(XXVIII) $[P\text{-}O\text{-}CH_2CH_2\text{-}CO\text{-}ON{=}CH\text{-}]_n\text{-}Z$;
(XXIX) $[P\text{-}O\text{-}CH_2CH_2\text{-}O\text{-}CO\text{-}ON{=}CH\text{-}]_n\text{-}Z$;
(XXX) $[P\text{-}O\text{-}CH_2CH_2\text{-}NH\text{-}CO\text{-}ON{=}CH\text{-}]_n\text{-}Z$;
(XXXI) $[P\text{-}O\text{-}CH_2CH_2\text{-}NH\text{-}CS\text{-}ON{=}CH\text{-}]_n\text{-}Z$;
(XXXII) $[P\text{-}O\text{-}CH_2CH_2\text{-}ON{=}CH\text{-}]_n\text{-}Z$;
(XXXIII) $[P\text{-}O\text{-}CH_2CH_2\text{-}NH\text{-}CO\text{-}CH_2\text{-}ON{=}CH\text{-}]_n\text{-}Z$;
(XXXIV) $[P\text{-}O\text{-}CH_2CH_2\text{-}O\text{-}CO\text{-}CH_2\text{-}ON{=}CH\text{-}]_n\text{-}Z$;
(XXXV) $[P\text{-}O\text{-}CH_2CH_2\text{-}CH(OH)\text{-}CH_2\text{-}ON{=}CH\text{-}]_n\text{-}Z$; and
(XXXVI) $[P\text{-}O\text{-}CH_2CH_2\text{-}CO\text{-}CH_2\text{-}ON{=}CH\text{-}]_n\text{-}Z$,

wherein P is a water-soluble polymer as previously described, Z represents a polypeptide, as described above, and n represents a number in the range 1 to X, where X is the maximum number of oxidation and activatable groups present in polypeptide Z. The carbon atom in the oxime linkage formed between the water-soluble polymer reagent and Z was originally present on Z, not the water-soluble polymer reagent.

Although polypeptides may be modified by the coupling of up to x water-soluble polymers per polypeptide molecule, it may be desirable to modify a given polypeptide by less than x water-soluble polymer molecules. It may be undesirable to derivatize a polypeptide with the maximum number of water-soluble polymers, i.e., x water-soluble polymers/polypeptide molecule, because for some polypeptides, increasing the number of water-soluble polymers per molecule of polypeptide may diminish biological activities as compared the unmodified polypeptide. For example see figures 2, 4, 5, 9, 10, and 11, for some results obtained with water-soluble polymer modified EPO.

Different methods of measuring the number of water-soluble polymer molecules attached to a glycoprotein molecule, as in hydrazone linked compounds of formulae X, XI, XII, XIII, XIV, XV, XVI, XVII, and XVIII and as in oxime linked compounds of formulae XXVIII, XXIX, XXX, XXXI, XXXII, XXXIII, XXXIV, XXXV and XXXVI may give different results. For the purpose of this application, when a polypeptide is said to be derivatized by a given number of water-soluble polymer molecules/molecule of protein, the number of water-soluble polymers given is the empirically determined figure measured by gel filtration chromatography retention time.

The synthesis of compounds of formulae X, XI, XII, XIII, XIV, XV, XVI, XVII, and XVIII may result in the creation of a mixture of reaction products differing from one another with respect to the exact number of water-soluble polymers attached to the polypeptide through hydrazone linkages and the sites on the polypeptide where these hydrazone linkages are present. Similarly, the synthesis of compounds of formulae XXVIII, XXIX, XXX, XXXI, XXXII, XXXIII, XXXIV, XXXV and XXXVI may result in the creation of a mixture of reaction products differing from one another with respect to the exact number of water-soluble polymers attached to the polypeptide through oxime linkage and the sites on the polypeptide where these oxime linkages are present. As the polymer P comprises multiple identical units of varying amounts, it will be appreciated that the molecular weight of P may vary considerably. Furthermore, when P is said to have a given molecular weight, that molecular weight may only be approximate, reflecting the average molecular weight of a population of molecules P differing with respect to one another in regards to the number of subunits present in the molecule. In general, P will have a molecular weight of about 200 to 200,000, preferably in the range of 700 to 30,000, more preferably in the range of 2,000-12,000. Suitable molecular weights for P, when the molecules of formulae I, II, III, IV, V, VI, VII, VIII, and IX and the molecules of formulae XXVIII, XXIX, XXX, XXXI, XXXII, XXXIII, XXXIV, XXXV and XXXVI are to be coupled to a polypeptide will vary in accordance with the specific polypeptide to be modified and the specific water-soluble polymer selected. Individual polypeptide molecules may be derivatized by one or more different water-soluble polymers by means of reaction with different embodiments of the compounds of formulae I, II, III, IV, V, VI, VII, VIII, and IX (the hydrazones), or the compounds of formulae XIX, XX, XXI, XXII, XXIII, XXIV, XXV, XXVI and XXVII (the oximes), or any combination of the hydrazones and the oximes.

An advantage of the subject invention is that polypeptides may be modified by the attachment of water-soluble polymers without substantially reducing the biological activity of the polypeptide, or reducing the biological activity to a lesser extent than the biological activity would be reduced by the attachment of a similar number of the same water-soluble polymers/polypeptide molecule by means of previously known chemical coupling methods and compounds. Aspects of the biological activity of EPO include the stimulation of red blood cell formation. A detailed description of the biological activity of EPO can be found in Krantz, S.B., Blood 77: 419-434 (1991).

Another advantage of the subject invention is that polypeptides modified by the compounds of formulae I, II, III, IV, V, VI, VII, VIII, and IX or the compounds of formulae XIX, XX, XXI, XXII, XXIII, XXIV, XXV, XXVI and XXVII may then retain a greater degree of their biological activity than when the same polypeptide is modified to the same degree by joining water-soluble polymers to polypeptides employing the frequently used (prior to

the subject invention) active esters of mPEG for lysine modification. Thus, the subject invention provides for modified polypeptides that possess the advantages associated with the covalent conjugation of water-soluble polymers while minimizing the loss of biological activity associated with the modification. Consequently, polypeptides that may be more highly derivatized by water-soluble polymers, and thus or otherwise possess the advantages associated with the higher degree of derivatization, may be produced that have the same level or a higher level of biological activity as polypeptides derivatized by water-soluble polymers to a lesser extent using conventional methodology.

Another advantage of using a hydrazone forming derivatives of PEG (and other water-soluble polymers) instead of PEG-amine for coupling PEG to a protein is that coupling of a hydrazide or oxylamine (or similar compounds) to an aldehyde yields a hydrazone or oxime respectively, while coupling through an amine gives an imine, which is less stable than a hydrazone or an oxime and needs to be reduced to give a stable derivative. Thus an extra step is required when using an amine instead of a hydrazone forming compound.

Another advantage of the subject invention is that higher levels of water-soluble polymers may be attached to glycoproteins than with other water-soluble polymer derivatives. The semicarbazide (formula III), thiosemicarbazide (formula IV), and carbonic acid dihydrazide (formula V) derivatives are of particular interest because of their higher reactivity than comparable hydrazide derivatives of the subject invention. Reactions involving the mPEG derivatization of EPO with semicarbazides, thiosemicarbazides and carboxylate hydrazide described herein have resulted the addition of up to about 31-34 mPEG molecules for each molecule of EPO, whereas similar reactions using corresponding hydrazide derivatives of EPO have resulted in the addition of about 6-12 molecules of mPEG to each molecule of EPO. Reactions between carbonic acid dihydrazide and hydrazide carboxylate derivatives and EPO have resulted in the addition of up to 22 mPEG molecules to each molecule of EPO. In order for hydrazide derivatives of mPEG to incorporate about 20 mPEG molecules to each molecules of EPO, very strong oxidation conditions were required, e.g., 50 mM periodate, 60 minutes incubation at room temperature. The subject mPEG semicarbazide and thiosemicarbazide derivatives could be used to provide EPO modified with PEG to a similar extent, but under more mild oxidation conditions, e.g., 10 mM periodate, for 5-15 minutes at 0°C. Strong oxidizing conditions may have an adverse effect on the structural and biological properties of many polypeptides, thus PEG semicarbazide, carbonic acid dihydrazide, hydrazide carboxylate, and thiosemicarbazide derivatives may be particularly useful compounds for modifying polypeptides with PEG (or other water-soluble polymers).

A novel series of oxylamine derivatives of mPEG have been synthesized and have been reacted to the oxidized carbohydrate groups of EPO. Some of the mPEG-oxylamines showed high reactivity to the oxidized carbohydrates. Also a lower number of mPEGs could be incorporated onto EPO and still give the high in vivo activity as seen with the semicarbazide and carboxylate hydrazide (hydrazone forming) mPEG-derivatives. This lower number for mPEG incorporation is advantageous in that shorter and milder oxidation conditions can be used in the modification. Also lesser amounts of mPEG-derivative can be used in the modification reaction.

Similarly, particularly high levels of water-soluble polymers are attached to glycoproteins when the compounds of formula XXI, formula XXIV, and formula XXIII are employed as compared to comparable formula XIX oxylamine derivative. Reactions involving derivatization of EPO with formula XXI and formula XXIV described herein have resulted in the addition of up to 18-19 mPEGS/EPO, and 31 mPEG molecules for every molecule of EPO, respectively, whereas similar reactions using corresponding formula XXII and XIX oxylamine derivatives of EPO have resulted in the addition of about 3-4 molecules of mPEG to each molecule of EPO. Perhaps more importantly, the bioactivity of resulting oxylamine-derivatized PEG-EPO is surprisingly high even at more moderate levels of attachment of water-soluble polymer to EPO (see hereinbelow and Figure 17). In this regard, bioactivity of a 12 mPEG isolated fraction of formula XXIII is of particular interest (See Figures 16 & 17).

The ability to generate long acting mPEG-EPO with high activity via coupling mPEG to the oxidized carbohydrate groups depends on the mPEG-derivative chosen. Some mPEG carbohydrate modifying derivatives are not reactive enough to attach an optimum amount of mPEG onto EPO. Some mPEG-derivatives require a high amount of incorporation onto EPO due to the stability of the resulting bond. An optimal amount of mPEG incorporation for the semicarbazide derivative is about 17-25, more preferably about 22; for the carboxylate hydrazide derivative, about 22-32, more preferably about 31. Reactivity of the mPEG-oxylamines is about 3-36 mPEGs/EPO.

The water-soluble polymer reagents of the subject invention may be used to modify a variety of polypeptides or similar molecules that contain aldehydes or functional groups with similar chemical reactivity, e.g., ketones, lactols, activated carboxylic acids or activated carboxylic acid derivatives, capable of chemically reacting with the hydrazide portion (or similar functional portion) of the subject water-soluble polymer reagent derived from the oxidation of hydroxyl groups, the oxidation of other oxidation activatable groups present on

the polypeptide of interest (including carbohydrate moieties when the polypeptide is a glycoprotein, and amino acid residues in the primary sequence, e.g., the N-terminus of serine, threonine, hydroxylysines), or hydrazine or oxylamine reactive group present on polypeptides prior to or after any oxidative treatment. Polypeptides of interest include antibodies, monoclonal and polyclonal, cytokines, growth factors, hormones, enzymes, protein or peptide ligands and the like. Polypeptides of interest for modification by hydrazone linkage or oxime linkage forming water-soluble polymer reagent molecules of the subject invention may be isolated from their natural sources, genetically engineered cells, e.g., CHO cells transformed with expression vectors for the production of EPO, or produced by various in vitro synthesis methods. A particularly preferred polypeptide for the purposes of the instant invention is EPO, and precursors, intermediates and mimetics thereof, whether human or recombinant.

While the water-soluble polymer reagents of the subject invention may be used to modify most polypeptides, it is of particular interest to modify (1) polypeptides for use as drugs, and (2) polypeptides for use in assays. Polypeptide for use in assays include specific binding proteins, polypeptides recognized by specific-binding proteins, and enzymes. By specific-binding proteins it is intended antibodies, hormone receptors, lectins, and the like.

Various polypeptides may be modified by the subject water-soluble polymer reagents and the subject methods for their use so as to be coupled to different water-soluble polymers and to differing degrees or modification. Varying parameters such as (1) the number of water-soluble polymers coupled to an individual polypeptide molecule, which will depend upon the reactivity of the derivatized mPEGs to the EPO, and the bioactivity of the resulting mPEG-EPO; e.g., reactivity from about 3-36 molecules of mPEG/EPO (2) the molecular weight of the water-soluble polymer, e.g., 2,000-12,000 daltons (3) the structure of the water-soluble polymer, e.g., monomethoxypoly(ethylene glycol) (4) the reaction conditions under which the reaction between the water-soluble polymer reagent and the polypeptide of interest, e.g., temperature and duration, and (5) the oxidation conditions under which the polypeptide for modification is activated for covalent conjugation, e.g., periodate at a concentration in the range of 10-40 μmol/mg of protein, may influence the biological properties of the resultant water-soluble polymer modified polypeptide.

In a preferred embodiment of the invention, activation of polypeptides for covalent conjugation is performed by mixing the protein for modification with periodate (0.1-1,000 μmole/mg protein) for a period of time in the range of one minute to three days, more preferably 0.5-50 μmole periodate/mg protein, for a time period in the range of 5 minutes to 180 minutes. In a preferred embodiment of the invention, activation for conjugation is performed by mixing the protein for modification with periodate at a temperature in the range of -10°-50°C, more preferably in the range of 0°-30°C.

In a preferred embodiment of the subject invention when the protein for modification is EPO, EPO is derivatized with the compounds of formulae II-VIII, more preferably the compounds of formulae II-V, the compound of formula III, the semicarbazide, and formula II, the carboxylate hydrazide, being particularly preferred, where the water-soluble polymer P is methoxypolyethylene glycol (mPEG) and each molecule of EPO is derivatized by 3-36, more preferably 17-25 molecules of methoxypolyethylene glycol (in the case of the semicarbazide), and more preferably 22-32 molecules of methoxypolyethylene glycol (in the case of the carboxylate hydrazide) and the mPEG used has an average molecular weight of about 5000 daltons. Preferred reaction conditions for the production of the mPEG5000 semicarbazide modified EPO are at 0-30°C, for 5 to 60 minutes, and 0.5-50 μmoles periodate/mg of EPO (with periodate as oxidizing agent). EPO for modification by the subject water-soluble polymer reagents and methods is preferably obtained from genetically engineered cells, more preferably from CHO cells genetically modified to produce EPO. By employing the preferred water-soluble polymer reagent and conditions for modifying EPO, unexpectedly prolonged biological half-life of EPO is obtained and increased hematocrit levels can be seen, for example, see Figures 2, 4 and 5.

In another preferred embodiment, EPO is derivatized with the compounds of formulae XX-XXVII, more preferably the compounds of formulae XXI, XXIV, and XXIII, wherein the water-soluble polymer P is mPEG and each molecule of EPO is derivatized by about 18-19 mPEGS, 31 mPEGS, and 17 mPEGs, respectively, for formulae XXI, XXIV and XXIII. These results were obtained when the mPEG used has an average molecular weight of about 5000 daltons. The compound of formula XXIII had a 12mPEG fraction, which had in vivo bioactivity comparable to 22mPEG semicarbazide and 31 mPEG carboxylate hydrazide. Reactivity (molecules of mPEG/EPO) for formulae XXI, XXIV and XXIII was in excess of that for PEG hydrazide under the same reaction conditions. Preferred reaction conditions for the production of the mPEG 5000 oxime may require more mild oxidation conditions such as a shorter oxidizing time or lower concentrations of oxidant than with the corresponding hydrazide to produce higher in vivo bioactivity.

The subject invention also provides methods of activating polypeptides for conjugation, i.e., covalent conjugation, with the subject water-soluble polymer reagents. These methods of activating polypeptides for conjugation comprise the step of partially oxidizing the polypeptides of interest. Partial oxidation may be achieved

by adding an oxidizing agent such as periodate and other oxidation agents known to those of skill in the art, or by adding an enzyme capable of catalyzing oxidation reactions on portions of the polypeptide of interest, e.g., galactose oxidase. The preferred method of partially oxidizing a polypeptide for activation for conjugation is by the addition of periodate in a concentration in the range of 0.1-1,000 μmole/mg protein, for a period of time in the range of one minute to three days, more preferably 0.5-50 μmole periodate/mg protein, for a time period in the range of 5 minutes to 180 minutes. The temperature at which the activation is performed is preferably in the range of -10°-50°C, more preferably in the range of 0°-30°C.

Salts of any of the macromolecules described herein, e.g., polypeptides, water-soluble polymers and derivatives thereof, will naturally occur when such molecules are present in (or isolated from) aqueous solutions of various pHs. All salts of polypeptides and other macromolecules having the indicated biological activity are considered to be within the scope of the present invention. Examples include alkali, alkaline earth, and other metal salts of carboxylic acid residues, acid addition salts (e.g., HCl) of amino residues, and zwitterions formed by reactions between carboxylic acid and amino residues within the same molecule.

The mode of administration of the preparations of the invention may determine the sites and/or cells in the organism to which the compound(s) will be delivered. The compounds of the invention can be administered alone but will generally be administered in admixture with a pharmaceutical carrier or diluent selected with regard to the intended route of administration and standard pharmaceutical practice. The preparations may be injected parenterally, for example, intra-arterially or intravenously. The preparations may also be delivered via oral, subcutaneous, or intramuscular routes. For parenteral administration, they can be used, for example, in the form of a sterile, aqueous solution which may contain other solutes, for example, enough salts or glucose to make the solution isotonic.

For the oral mode of administration, the EPO compositions of the invention can be used in the form of tablets, capsules, lozenges, powders, syrups, elixirs, aqueous solutions and suspensions and the like. In the case of tablets, carriers which can be used include lactose, sodium citrate, and salts of phosphoric acid. Various disintegrants such as starch, and lubricating agents such as magnesium stearate are commonly used in tablets. For administration in capsule form, useful diluents are lactose and high molecular weight polyethylene glycols. When aqueous solutions are required for oral use, certain sweetening and/or flavoring agents can be added.

For administration to humans in the treatment of disease states responding to EPO therapy, the prescribing physician will ultimately determine the appropriate dosage for a given human subject, and this can be expected to vary according to the weight, age and response of the individual as well as the nature and severity of the patient's disease. The dosage of the drug in pegylated form may generally be about that employed for native drug, however, it may in some cases be preferable or necessary to administer dosages outside these limits.

It is also of interest to supply the water-soluble polymer reagents of formulae I, II, III, IV V, VI, VII, VIII, and IX, and XIX, XX, XXI, XXII, XXIII, XXIV, XXV, XXVI and XXVII separately or in various combinations, in the form of a kit, so as to provide for the convenient and reproducible derivatization of polypeptides of interest. Kits of interest may contain solutions comprising the water-soluble polymer reagent of formulae I, II, III, IV, V, VI, VII, VIII, or IX, or those of formulae XIX, XX, XXI, XXII, XXIII, XXIV, XXV, XXVI, XXVII, buffers, oxidizing agents, reaction indicator compounds, protein concentration measurement reagents, e.g., for Bradford assays, and the like. Compounds included in kits are preferably provided in pre-measured portions and pre-mixed solutions so as to provide for reproducibility and minimize error. Kits also preferably contain instructions. Instructions are directed to various steps in performing the subject methods.

## SYNTHESIS OF WATER-SOLUBLE POLYMER DERIVATIVES

The following syntheses of the subject compounds are exemplary and are not included for the purpose of limiting the invention. The person of average skill in the art of organic chemistry can devise variations on the exemplified syntheses.

## SYNTHESIS OF HYDRAZONE FORMING m-PEG

### Synthesis of mPEG-Hydrazide

There are several ways to synthesize mPEG-hydrazide. Two methods are presented.

mPEG5000-acid (20.8 g, 4 mmol) was dissolved in 30 ml dichloromethane and t-butyl carbazate (2.64 g, 8 mmol) in 15 ml dichloromethane was added followed by 1.68 g (8 mmol) dicyclohexylcarbodiimide which was dissolved in 10 ml dimethylformamide. After running the reaction over night at room temperature the reaction

mixture was filtered. The filtrate was concentrated, and the resulting residue was taken up in dichloromethane. Ether was added to precipitate the mPEG-t-butyl-carbazide which was filtered and dried. The product was placed in a dicholormethane/ trifluoroacetic acid (1:1) mixture. After 40 minutes the solution was concentrated, redissolved in dicholormethane and ether was added. The product was recovered by filtration. Yield 17.7 g. IR: (C=O) 1730, 1700. Analysis. Calcd. for N, 0.55. Found: N, 0.44.

An alternative method shown below converts mPEG-alcohol to an ester. The mPEG-ester then is hydrolyzed with hydrazine to give mPEG-hydrazide. The synthesis of mPEG-ester is similar to the procedure of Royer, G.P., and Anantharmaiah, G.M. (1979) J. Amer. Chem. Soc. 101, 3394-3395.

NaH

mPEG-OH --------------------->$mPEG-O-CH_2-CO_2C_6H_5$

$Br-CH_2-CO_2C_6H_5$

$H_2NNH_2$

$mPEG-O-CH_2-CO_2C_6H_5$ ----------->$mPEG-O-CH_2-CO-NHNH_2$

In a typical synthesis mPEG-OH was dried for about five hr at 85° in a high vacuum oven. After cooling 5 g mPEG-OH (MW = 5000, 1 mmol) was dissolved in 5 ml dry tetrahydrofuran. To 26.4 mg (1.1 mmol) sodium hydride was added 1 ml dry tetrahydrofuran. The mPEG5000 solution was added to the NaH dropwise. The mixture was stirred for one hr at room temperature in an argon atmosphere. During this time the solution became orange in color. Bromoacetyl acid benzyl ester (2.29 g, 10 mmol) was dissolved in 1 ml dry tetrahydrofuran, and this solution was added dropwise to the mPEG5000 mixture. The reaction was stirred overnight at room temperature under an argon atmosphere after which time it was filtered. Cold ether was added to the filtrate to precipitate the mPEG5000-benzyl ester, and the solid was collected and dried. Yield 4.5 g. IR (C=O) 1752. The compound was further purified by gel filtration on a LH-20 column eluting with methanol/methylene chloride (5:1).

The mPEG5000-benzyl ester was converted to the hydrazide by treatment with hydrazine. In a typical experiment 1.0 g mPEG5000-benzyl ester (0.194 mmol) was dissolved in 3 ml methanol/methylene chloride (5:1) in an argon atmosphere. Hydrazine (0.091 ml, 2.91 mmol) was added and the solution was stirred at room temperature for around 70 hr. The mixture was placed on a LH-20 column eluting with methanol/methylene chloride (5:1). The mPEG5000-hydrazide was separated from the hydrazide and was precipitated with ether. The solid was collected by filtration. Yield 0.78 g. IR ($H_2N$-C=O): 1669. Analysis. Calcd. for N, 0.55. Found: N, 0.34.

Also synthesized were mPEG2000-hydrazide, mPEG6000-hydrazide, mPEG8500-hydrazide, and mPEG12000-hydrazide using the procedures described above.

Synthesis of mPEG-Hydrazine Carboxylate

$H_2NNH_2$

mPEG-O-CO-Im ----------------> $mPEG-O-CO-NHNH_2$

The above mPEG5000-hydrazine carboxylate was synthesized as follows. Methoxypolyoxyethylene imidazolyl carbonyl (from Sigma Chemical, 2.5 g, 0.49 mmol) was treated with hydrazine ( 0.077 ml, 2.45 mmol) in 10 ml methylene chloride. After 4 hr at room temperature the reaction mixture was filtered, and the filtrate was treated with cold ether. The resulting precipitate was collected. Yield 2.22 g. IR (C=O): 1718. Analysis. Calcd. for N, 0.55. Found: N, 0.595.

Synthesis of mPEG-Semicarbazide

$COCl_2$

$mPEG-NH_2$ --------------------> mPEG-NH-CO-Cl

$$\text{mPEG-NH-CO-Cl} \xrightarrow{H_2NNH_2} \text{mPEG-NH-CO-NHNH}_2$$

The above mPEG5000-semicarbazide was synthesized as follows. mPEG5000-amine was synthesized as described by Rajasekharan Pillai, V.N., and Mutter, M. (1980) J. Org. Chem. 45, 5364-5370. The mPEG5000-amine (2 g, 0.4 mmol) was dissolved in 9 ml dichloromethane and 0.28 ml triethylamine was added. To the mixture in an argon atmosphere was added phosgene (in toluene, 0.42 ml, 0.8 mmol). The reaction went overnight and then was bubbled with argon to remove any excess phosgene. The solution was concentrated, and the residue was dissolved in dicchloromethane and 0.063 ml hydrazine (2 mmol) was added followed by 2 ml methanol. The reaction went for 4 hr after which time cold ether was added, and the precipitate was removed by filtration and dried. Yield 1.51 g. IR (C=O): 1683. Analysis. Calcd. for N, 0.83. Found: N, 0.56.

Also synthesized were the semicarbazides of mPEG2000, mPEG6000, mPEG8500, and mPEG12000 using the procedures described above.

Synthesis of mPEG-Thiosemicarbazide

To 1.5 g mPEG5000-amine (0.3 mmol) in 5 ml dichloromethane was added 0.1 ml triethylamine (0.75 mmol) and 0.071 g (0.3 mmol) di-2-pyridylthionocarbonate. The reaction went overnight, where upon 0.047 ml (0.3 mmol) hydrazine was added. After 4 hr the mixture was filtered, and the filtrate was treated with cold ether. The product was collected by filtration. Yield 1.34 g. IR (N-H stretch): 3332. Analysis. Calcd. for N, 0.83. Found: N, 0.255.

Synthesis of mPEG-Carbonic Acid Dihydrazide

In a reaction flask purged with argon was added t-butyl carbazate (0.04 g, 0.3 mmol) in 2 ml dichloromethane, 0.084 ml triethylamine (0.6 mmol), and 0.03 g triphosgene (0.1 mmol). After 5 minutes 1.5 g mPEG5000-amine (0.3 mmol) in 4 ml dichloromethane was added. The reaction went overnight after which time cold ether was added to precipitate the product. The product was isolated by filtration. Yield 1.44 g. The protected dihydrazide (0.61 g) was treated with 2 ml trifluoroacetic acid at room temperature for 10 minutes. The trifluoroacetic acid was removed, and the resulting oil was dissolved in methylene chloride and concentrated. This step was repeated. The oil was dissolved in methylene chloride and the product was precipitated with cold ether. The product was isolated by filtration. Yield 0.41 g. IR (C=O):1695. Analysis calcd. for N, 1.37. Found: 0.41.

Synthesis of mPEG-Arylhydrazide

$$\text{mPEG-NH}_2 \xrightarrow{\text{Boc-NHNH-}C_6H_4\text{-COOH}} \text{mPEG-NH-CO-}C_6H_4\text{-NHNH}_2$$

The above mPEG5000-arylhydrazine was synthesized as follows. Boc-NHNH-$C_6H_4$-COOH was prepared by reacting 4-hydrazinobenzoic acid with di-tert-butyl pyrocarbonate in dioxane in the presence of base at 0°C. The protected aryl acid hydrazine (0.378 g, 1.5 mmol) was reacted with mPEG-amine (1.5g, 0.3 mmol) in a dichloromethane/dimethylformamide solution (4ml, 1:1). Also added was dicyclohexylcarbodiimide (0.31g, 1.5 mmol), 1-hydroxybenzotriazole (0.2g, 1.5 mmol), and triethylamine (0.21ml, 1.5mmol). The reaction went overnight, after which time the contents were filtered. The filtrate was treated with ether, and the precipitate was collected. The precipitate was treated with trifluoroacetic acid, and after 30 min, the trifluoroacetic acid was removed. Ether was added to the oily residue to precipitate the mPEG-arylhydrazine product. The product was isolated by filtration. Yield 1.19g. IR (N-H): 3267; (C=O): 1655; (C=C): 1606. Analysis. Calcd. for N, 0.82. Found: N, 0.50.

## SYNTHESIS OF OXIME-FORMING m-PEG

### Synthesis of $CH_3O-(CH_2CH_2O)_n-CH_2CH_2-CO-ONH_2$

1. BocNHOH/TEA

$$mPEG-CO-NHS \xrightarrow{\text{1. BocNHOH/TEA; 2. TFA}} mPEG-O-CH_2CH_2-CO-ONH_2$$

2. TFA

mPEG-5000-succinimide ester (NHS) (2.0g, 0.4 mmol) was dissolved in 10ml dichloromethane. t-Butyl N-hydroxycarbamate (0.53g, 4 mmol) was added followed by 0.7ml triethylamine (5 mmol). After running the reaction overnight cold ether was added, and the resulting precepitate was collected by filtration, washed, and dried. The product (1.5g) was placed in a dicholormethane/trifluoroacetic acid (1:1) mixture. After 60 minutes the solution was concentrated. The compound was further purified by gel filtration on a LH-20 column eluting with methanol/methylene chloride (5:1). Yield 1.2g IR: (C=O) 1741. Analysis Calcd. for N,0.28. Found: N,0.13.

### Synthesis of $CH_3O-(CH_2CH_2O)_n-CH_2CH_2-O-CO-ONH_2$;

1. BocNHOH/TEA

$$mPEG-O-CO-Im \xrightarrow{\quad} mPEG-O-CH_2CH_2-O-CO-ONH_2$$

2. TFA

mPEG-5000-oxycarbonylimidazole (2.0g, 0.4 mmol) was dissolved in 10ml dichloromethane. t-Butyl N-hydroxycarbamate (0.53g, 4 mmol) was added followed by 0.7ml triethylamine (5 mmol). After running the reaction overnight cold ether was added, and the resulting precipitate was collected by filtration, washed, and dried. The product (1.5g) was placed in a dichloromethane/trifluoroacetic acid (1:2) mixture. After 60 minutes the solution was concentrated. The compound was further purified by gel filtration on a LH-20 column eluting with methanol/methylene chloride (f:1). Yield 1.2g. IR:(C=O) 1692. Analysis Calcd. for N, 0.28. Found: N, 0.15.

### Synthesis of $CH_3O-(CH_2CH_2O)_n-CH_2CH_2-NH-CO-ONH_2$

1. Im-CO-Im

$$mPEG-NH_2 \xrightarrow{\quad} mPEG-O-CH_2CH_2-NH-CO-ONH_2$$

2. BocNHOH/TEA
3. TFA

mPEG-5000-amine (2.0g, 0.4 mmol) was dissolved in 10ml chloroform along with carbonyldimidazole (o.23gm, 1.45 mmol). This reaction followed the procedure for activation of mPEG-alcohol with carbonyldimidazole as described by Ranucci, E., and Feruti, P. (1991) Macromolecules 24, 3747-3752. The reaction was stirred for two hours at room temperature after which time 7ml water was added, and the organic layer was extracted. The water extraction was repeated five times. The organic layer was dried over sodium sulfate, and the salt was filtered. Added to the filtrate was t-butyl N-hydroxycarbamate (0.53g, 4 mmol) along with 0.7ml triethylamine (5 mmol). The reaction was stirred overnight after which time cold ether was added, and the resulting precipitate was collected by filtration, washed, and dried. The product (1.5g) was placed in a dicholormethane/trifluoroacetic acid (1:2) mixture. After 60 minutes the solution was concentrated. The compound further purified by gel filtration on a LH-20 column eluting with methanol/methylene chloride (5:1). Yield 1.3g. IR: (C=O):1726. Analysis. Calcd. for N, 0.55. Found: N, 0.43.

Synthesis of $CH_3O-(CH_2CH_2O)_n-CH_2CH_2-NH-CS-ONH_2$;

1. $(C_5H_4NO)_2CS$

$mPEG-NH_2$ ---------------> $mPEG-O-CH_2CH_2-NH-CS-ONH_2$

2. BocNHOH/TEA

3. TFA

mPEG-5000-amine (1.5g, 0.3 mmol) was dissolved in 30ml dichloromethane. Triethylamine (0.1ml, 0.75 mmol) was added followed by di-2-pyridylthionocarbonate (0.082g, 0.35 mmol). The reaction was stirred for two hours at room temperature after which time t-butyl N-hydroxycarbamate (0.53g, 4 mmol) was added along with 0.5ml triethylamine (3.75 mmol). The reaction was stirred overnight after which time cold ether was added, and the resulting precipitate was collected by filtration, washed, aNd dried. The product (1.6g) was placed in dicholormethane/trifluoroacetic acid (1:1) mixture. After 30 minutes the solution was concentrated. The compound was further purified by gel filtration on a LH-20 column eluting with methanol/methylene chloride (5:1). Yield 0.72g. IR: (C=S) : 1684. Analysis. Calcd. for N, 0.55. Found: N, 0.30.

Synthesis of $CH_3O-(CH_2CH_2O)_n-CH_2CH_2-ONH_2$

1. BocNHOH/TEA

mPEG-O-Trs ---------------> $mPEG-O-CH_2CH_2-ONH_2$

2. TFA

mPEG-5000-tresylate (2.0g, 0.4 mmol) was dissolved in 10ml dichloromethane to which t-butyl N-hydroxycarbamate (0.53g, 4 mmol) and 0.7ml triethylamine (5mmol) were added. The mixture was heated to 45° (reflux), and the reaction ran overnight. Cold ether was added to the reaction mixture, and the resulting precipitate was collected by filtration, washed, and dried. The product (1.5g) was placed in a dicholormethane/trifluoroacetic acid (3:7) mixture. After 60 minutes the solution was concentrated. The compound was further purified by gel filtration on a LH-20 column eluting with methanol/methylene chloride (5:1). Yield 1.6g. Analysis. Calcd. for N, 0.28. Found: N, 0.19.

An alternative synthesis is the following. t-Butyl N-hydroxycarbamate (0.53g, 4 mmol) was placed in lml tetrahydrofuran followed by NaH (78 mg, 3.25 mmol). After a few minutes this solution was added to mPEG-5000-tresylate (1.0g, 0.2 mmol) which was in 5ml tetrahydrofuran. The mixture was heated to 40°, and the reaction went overnight. Cold ether was added to the reaction mixture, and the resulting precipitate was collected by filtration, washed and dried. the product (1.5g) was placed in a dicholormethane/trifluoroacetic acid (3:7) mixture. After 60 minutes the solution was concentrated. The compound was further purified by gel filtration on a LH-20 column eluting with methanol/methylene chloride (5:1). Yield 0.75g. Analysis. Calcd. for N, 0.28. Found: N, 0.10.

Synthesis of $CH_3O-(CH_2CH_2O)_n-CH_2CH_2-NH-CO-CH_2-ONH_2$

1. $BocNHOCH_2COOH$

PyBOP/DIEA

$mPEG-NH_2$ ---------------> $mPEG-O-CH_2CH_2-NH-CO-CH_2ONH_2$

2. TFA

mPEG-5000-amine (2.0g, 0.4 mmol) and Boc- aminoxyacetic acid (0.2g, 1.05 mmol) were dissolved in 20ml dichloromethane. Benzotriazol-1-yl-oxytripyrrolidinophosphonium hexafluorophosphate (1.1g, 2mmol) was added followed by diisopropylethylamine (0.7ml, 3.9 mmol). The reaction was stirred for about 72 hours at room temperature after which time cold ether was added, and the resulting precipitate was collected by filtration, washed, and dried. Half of the collected precipitate was paced in a dicholormethane/trifluoroacetic acid (3:7) mixture. After 60 minutes the solution was concentrated. The compound was further purified by gel filtration on a LH-20 column eluting with methanol/methylene chloride (5:1). The resulting yellow precipitate was taken up in water and was treated with decolorizing carbon. After about 24 hr. the decolorizing carbon was

filtered, and the clear filtrate was concentrated. The residue was dissolved in dichloromethane, dried with sodium sulfate, filtered, and the filtrate was treated with cold ether. A white product was obtained. Yield 0.5g. IR: (C=0): 1676. Analysis. Calcd. for N, 0.55. Found: N, 0.50.

Synthesis of $CH_3O\text{-}(CH_2CH_2O)_n\text{-}CH_2CH_2\text{-}O\text{-}CO\text{-}CH_2\text{-}ONH_2$

```
                1. BocNHOCH2COOH
                   PyBOP/DIEA/DMAP
mPEG-OH        ----------------> mPEG-O-CH2CH2-O-CO-CH2ONH2
                2. TFA
```

mPEG-5000-alcohol (2.0g, 0.4 mmol) and Boc- aminooxyacetic acid (0.2g, 1.05 mmol) were dissolved in 20 ml dichloromethane. Benzotriazol-1-yl-oxytripyrrolidinophosphonium hexaflurophosphate (1.1g, 2mmol) was added followed by diisopropylethylamine (0.7ml, 3.09 mmol). The reaction was stirred for about 2 hours at room temperature after which time dimethylaminopyridine (0.244g, 2 mmol) was added. After about four days cold ether was added, and the resulting precipitate was collected by filtration, washed, and dried. The precipitate was taken up in water and was treated with decolorizing carbon. After about 24 hr. the decolorizing carbon was filtered, and the clear filtrate was concentrated. The residue was dissolved in dichloromethane, dried with sodium sulfate, filtered, and the filtrate was treated with cold ether. Analysis. Calcd. for N, 0.28. Found: N, 0.14.

Half of the collected precipitate was placed in a dicholormethane/trifluoroacetic acid (2:1) mixture. After 30 minutes the solution was concentrated. The compound was further purified by gel filtration on a LH-20 column eluting with methanol/methylene chloride (5:1). Yield 0.3g. IR: (C=0): 1734.

Synthesis of $CH_3O\text{-}(CH_2CH_2O)_n\text{-}CH_2CH_2\text{-}CH(OH)\text{-}CH_2\text{-}ONH_2$

```
          O    1. BocNHOCH2COOH
mPEG-CH-CH2---------------> mPEG-O-CH2CH2-CH(OH)-CH2-ONH2
               2. TFA
```

mPEG-5000-epoxide (1.0g, 0.2 mmol) was dissolved in 10ml 0.1M NaOH. t-Butyl N-hydroxycarbamate (0.53g, 4 mmol) was added. After running the reaction overnight the reaction mixture was extracted with dichloromethane. Sodium sulfate was added and was filtered. Cold ether was added the dichloromethane solution, and the resulting precipitate was collected by filtration, washed, and dried. The compound was further purified by gel filtration on a LH-20 column eluting with methanol/methylene chloride (5:1). The protected mPEG-derivative (0.25g) was placed in a dicholormethane/trifluoroacetic acid (1:1) mixture. After 30 minutes the solution was concentrated and taken up in dichloromethane. The compound was isolated by precipitation from ether. Yield 0.2g. IR: (O-H): 3447. Analysis. Calcd. for N, 0.28. Found: N, 0.21.

Synthesis of $CH_3O\text{-}(CH_2CH_2O)_n\text{-}CH_2CH_2\text{-}CO\text{-}CH_2\text{-}ONH_2$

```
                                      1. DMSO/Ac2O
mPEG-O-CH2.CH2-CH(OH)-CH2-ONH-Boc     ----------->
                                      2. TFA
   mPEG-O-CH2CH2-CO-CH2-ONH2
```

mPEG-O-$CH_2CH_2$-CH(OH)-$CH_2$-ONH-Boc (0.3g, 0.6 mmol) was placed in 3 ml dry dimethyl sulfoxide followed by the addition of 2 ml dry acetic anhydride. The reaction went for about 24 hr. at room temperature after which time cold ether was added. The resulting precipitate was collected by filtration, washed and dried. The product was placed in a dicholormethane/trifluoroacetic acid (1:1) mixture. After 30 minutes the solution was concentrated. The compound was isolated by precipitation from cold ether. Yield 0.18g. IR: (C=0): 1698. Analysis. Calcd. for N, 0.28. Found: N, 0.20.

Synthesis of mPEG-Ornithine Semicarbazide

mPEG-5000-amine (5.0 g, 1. mmol) was dissolved in 5 ml dry methylene chloride and 10 ml dry dimethylformamide was added. Fmoc-Orn(Boc)-OPfp (3.1 g, 5 mmole) was added. After 1 hr. at room temperature the solution was concentrated. Water was added to the residue, and the solution was filtered, centrifugred, and filtered to remove the dispersed solid in the aqueous solution. The filtered aqueous solution was concentrated, and the residue was taken up in methylene chloride and was dried with sodium sulfate. The solution was filtered. The filtrate was treated with cold ether. The resulting precipitate was colleced by filtration, washed, and dried. Yield 3.3 g. IR: (C=0):1713, 1680. The Fmoc group was removed by treating the compound with 25% piperidine (in methylene chloride) for 30 min. Cold ether was added to the solution to precipitate the mPEG-derivative. The precipitate was collected, washed, and dried. The free alpha amino group was acetylated by dissolving 1.4 g of the mPEG-derivative with 3 ml methylene chloride and adding 1 ml acetic anhydride. After about 1.7 hr. at room temperature the solution was concentrated. The resulting solid was treated with trifluoroacetic acid in methylene chloride (3:5) for 1 hr. at room temperature. The solvent was removed and resulting oil was taken up in methylene chloride and cold ether was added. The precipitate formed was collected, washed, and dried. Yield 1.1 g. IR: (C=O): 1685. The mPEG-derivative was dissolved in 3 ml dry methylene chloride and triethylamine (0.54 ml, 3.84 mmole) was added followed by 1 ml phosgene in toluene (1.92 mmole) and an additional 2 ml dry methylene chloride. The reaction went overnight at room temperature after which time the solvent was removed. The residue was dissolved in 3 ml dry methylene ch loride and 0.2 ml hydrazone (5.76 mmole) was added. Dry methanol was added until the solution became clear (3.4 ml). After 4 hr. at room temperature the solution was clarified by centrifugation and was concentrated. The compound was purified by gel filtration on a LH-20 column eluting with methanol/methylene chloride (5:1). Yield 0.7 g. IR: (C=O): 1675.

The invention having been described, the following examples are offered by way of illustration, not by way of limitation, of the subject invention.

EXAMPLES

Modification of EPO (Hydrazide Method)

In a typical experiment, EPO (0.5-1.0 mg) (obtained from Ortho Biotech) was placed in 100 mM sodium acetate, pH 5.6, total volume 0.786 ml. Enough 10 mg/ml solution of sodium periodate was added to give a final concentration of sodium periodate at 10 mMol. The oxidation went for 30 min at 0°C in the dark after which time 0.33 ml 80 mMol $Na_2SO_3$ was added. After 5 min the solution was concentrated and washed three times with 100 mMol sodium acetate, pH 4.2 in a microconcentrator. After the final concentration the oxidized EPO solution was brought up to 1.0 ml with 100 mM sodium acetate. mPEG5000-hydrazide (50 mg) was added to the oxidized EPO. The mixture was stirred over night at room temperature. The mPEG5000-EPO was purified by gel filtration using a Sephacryl S-200-HR column (1 mm x 45 mm) eluting with a phosphate buffer containing 0.05% sodium azide. The amount of mPEG modifying EPO was determined by HPLC gel filtration using a either a Zorbax® GF-250 or GF-450 column using a 0.1 M phosphate buffer, pH 7. From 6-12 molecules mPEGs were found to be attached to each molecule of EPO.

Modification of EPO (Semicarbazide Method)

The same procedure as above for the hydrazide method was performed, except the reaction time for oxidation was decreased to 5 minutes and a decreased amount of mPEG-semicarbazide (10 mg) was used compared to the amount of mPEG-hydrazide (50 mg). Even with the decreased oxidation time and less mPEG added, more (about 18) mPEG molecules were attached to EPO. If longer oxidation times (15 min) and more mPEG-semicarbazide is added, around 30 mPEG molecules can be attached to EPO depending on the molecular weight of mPEG used. Thus mPEG-semicarbazide appears to be more reactive than mPEG-hydrazide and attaches many more mPEG molecules to EPO than mPEG-hydrazide is able to under similar reaction conditions.

A comparison of the effect of modifying EPO with mPEG on either the carbohydrate groups or on the amino acid side chains is shown in Fig. 1. Analytical HPLC gel filtration conditions are the same as described above. The chromatogram of unmodified EPO is presented in Fig. 1a. A single peak with a retention time of 10.5 min is found. When EPO is modified with mPEG5000 on its carbohydrate groups (Fig. 1b), a single large peak with a retention time of 9.4 minutes is seen for the unpurified reaction product. When EPO is reacted with a succinimide ester of mPEG5000 which reacts with the side chain of lysine, a heterogeneous mixture of reaction products is obtained (peaks from 7.5 - 10.2 minutes). A similar heterogeneous pattern for mPEG modification

using succinimide coupling to CSF-1, interleukin-2, and β-interferon has been found (U.S. Pat No. 4,847,325 and 4,9117,888). There are also more low molecular weight impurities present with succinimide coupling (Fig. 1c). Active ester coupling using succinimide derivatives of mPEG has been the preferred method for attaching mPEG to proteins [Nucci, M.L., Shorr, R., and Abuchowski, A. (1991) Adv. Drug Delivery Rev., 6, 133-151]. EPO was also derivatized with mPEG8500 using the above-described semicarbazide method, see Figure 4 for results of biological experiments. The above-described semicarbazide method was also used to obtain EPO modified with mPEG12000 (see Figure 3) and EPO modified with mPEG2000 (see Figure 10).

EPO also was modified with thiosemicarbazide, hydrazide carboxylate, and carbonic acid dihydrazide derivatives of mPEG. These derivatives of mPEG performed like the semicarbazide derivatives of mPEG in that high levels of coupling mPEG to EPO could be obtained using these derivatives when compared to the hydrazide derivatives of mPEG. Other conditions for the oxidation of EPO can be used such as increased temperature, increased concentration of sodium periodate, and increased or decreased reaction times as long as these oxidation conditions do not impair the biological activity of EPO.

Large-Scale Modification of EPO (Semicarbazide or Carboxylate Hydrazide Method)

EPO (12.0mg) (obtained from Ortho Biotech) was placed in 100 mM sodium acetate, pH 5.5, total volume 1.8 ml. Sodium periodate (0.215 ml) at a concentration of 40 mg/ml was added. The oxidation went for 20 minutes at 0°C in the dark after which time 0.02 ml of ethylene glycol was added, and the admixture stirred for 10 minutes at 0°C. The oxidized-EPO was purified by gel filtration using a Sephadex® G-25 column (2.5 cm X 9 cm) and eluted with 100mM sodium acetate buffer, pH 4.3. Eluted oxidized EPO (10-11ml) was pooled. mPEG5000 semicarbazide (100 mg) was added to the purified oxidized EPO. The mixture was stirred overnight at room temperature. The mPEG-5000 EPO was purified by gel filtration using a Sephacryl® S-200-HR column eluting eith buffer consisting of 0.2M NaCl, 0.02 M sodium citrate, 0.025% sodium azide, pH 7.0.

The above modification was repeated using 200 mg of mPEG5000 semicarbazide. Reactivity was about 22 mPEG molecules per molecule of EPO.

The above modification was repeated employing half the amount of the reactants as specified hereinabove, using 200 mg of carboxylate hydrazide. Reactivity was about 30 mPEG molecules per molecule of EPO.

Modification of EPO (Oxime Method)

A. mPEG-$CH_2CH_2$-NH-CO-$CH_2$-$ONH_2$

The same procedure as above for the large-scale semicarbazide method was performed. However, instead of addition of mPEG5000 semicarbazide, mPEG5000-CH2-CH2-NH-C0-$CH_2$-$ONH_2$ (50 mg) was admixed with 2.15 ml of oxidized EPO at room temperature overnight. The mPEG5000-EPO was purified by gel filtration using a Sephacryl® S-200-HR column eluting with buffer consisting of 0.2M NaCl, 0.02 M sodium citrate, 0.025% sodium azide, pH 7. About 31 molecules of mPEG5000 were found to be attached to each molecule of EPO as determined by HPLC gel filtration using a Phenomenex Biosep-Sec-S4000 column (30 cm X .017 cm). A minor fraction consisting of 25 molecules of mPEG-EPO was also isolated, and was used for biological testing. See Figures 16, 18 and 19.

B. mPEG-O-$CH_2CH_2$-NH-CO-$ONH_2$

The above modification of EPO was repeated using mPEG-O-$CH_2CH_2$-NH-CO-$ONH_2$. About 18-19 molecules of mPEG5000 were found to be attached to each molecule of PEG as determined using the methods as in modification A above. Biological data is shown in Figures 16, 18 and 19.

C. mPEG-O-$CH_2CH_2$-$ONH_2$

The above modification of EPO was repeated using mPEG-O-$CH_2CH_2$-$ONH_2$. About 17 molecules of mPEG5000 were found to be attached to each molecule of PEG as determined using the methods as in modification A above. Two minor fractions of 22mPEG, 12mPEG were also isolated(Figure 17, 18, 19).

D. mPEG-O-$CH_2CH_2$-CO-$ONH_2$

The above modification of EPO is repeated using the PEG-oxime derivative mPEG-O-$CH_2CH_2$-CO-$ONH_2$. About 3 molecules of mPEG5000 were found to be attached to each molecule of EPO, as determined using

the methods as for modification A above.

E. $mPEG-O-CH_2CH_2-CH(OH)-CH_2-ONH_2$

The above modification of EPO is repeated using the PEG-oxime derivative $mPEG-O-CH_2CH_2-CH(OH)-CH_2-ONH_2$. About 31 molecules of mPEG5000 were found to be attached to each molecule of EPO, as determined using the methods as for modification A above.

F. $mPEG-O-CH_2CH_2-NH-CS-ONH_2$

The above modification of EPO is repeated using the PEG-oxime derivative $mPEG-O-CH_2CH_2-NH-CS-ONH_2$. About 4 molecules of mPEG5000 were found to be attached to each molecule of EPO, as determined using the methods as for modification A above.

Biological Activity of mPEG-EPO

The mPEG-EPO derivatives were assayed for biological activity in vivo by measuring the increase in erythrocytes generated after injection of the modified protein (Egrie, J.C., Strickland, T.W., Lane, J., Aoki, K., Cohen, A.M., Smalling, R., Trail, G., Lin, F.K., Browne, J.K., and Hines, D.K. (1986) Immunobiol. 172: 213-224). Briefly, mice (female CD-1, eight weeks old) were injected either intraperitoneally or subcutaneously with 0.4 μg protein once a day for two consecutive days. Blood was withdrawn on predetermined days for hematocrit readings.

The in vivo biological activities (hematocrit levels) of the mPEG-EPOs linked via hydrazide derivatives of mPEG is presented in Figures 2, 4 and 5 and Tables I and II. To summarize the findings, Figures 2 and 5 show that the optimal number of mPEG coupling is not obvious and has to be determined by synthesis and biological testing in order to give the best mPEG-EPO. Figure 4 compares mPEG coupling using hydrazide and semicarbazide linkers. Higher and longer hematocrit levels for mPEG-EPO could be obtained using the semicarbazide linker. The observed results are due, in part, to the higher level of incorporation of mPEG which could be obtained using the semicarbazide linker. Table I shows the biological activity of the mPEG-EPOs as a function of the number of mPEGs incorporated and the molecular weight of mPEG used.

Table I summarizes the biological activity of different hydrazide mPEG-EPOs comparing molecular weight of mPEG used and the amount of mPEG coupled.

Table II shows a comparison of the different mPEG hydrazide linkers used. Table II summarizes the biological activities of EPO modified with different mPEG5000-hydrazide derivatives where optimal amounts of mPEG were incorporated. Not all the carbohydrate mPEG-derivatives give the same biological activity when coupled to EPO due to the inability to sufficiently couple an optimal amount of mPEG or other factors. Biological activities are the best values obtained for each linker.

TABLE I

Table I: Biological Activity of Different mPEG-EPOs

| #mPEGs Coupled * | Molecular Weight mPEG | Max. Hematocrit (%)** | Duration of Activity Relative to EPO (Days)*** |
|---|---|---|---|
| 15 | 2000 | 60 | 14 |
| 6 # | 5000 | 53 | 8 |
| 12 # | 5000 | 51 | 7 |
| 18 | 5000 | 61 | 19 |
| 22 | 5000 | 61 | 20 |
| 24 | 5000 | 58 | 13 |
| 28 | 5000 | 54 | 14 |
| 17 | 6000 | 59 | 16 |
| 12 # | 8500 | 54 | 7 |
| 20 | 8500 | 58 | 14 |
| 34 | 8500 | 47 | 7 |
| 6 # | 12000 | 54 | 7 |
| 14 | 12000 | 53 | 11 |
| 29 | 12000 | 51 | 7 |

* As Determined by Size Exclusion Chromatography

** Biological Assay Described in Experimental Section

*** Days from EPO Maximum Hematocrit Level (48 on Day 4) Required to Reach EPO's Maximum Hematocrit Level After Attaining its Own Maximum Hematocrit Level

\# Hydrazide Linker Used. All Others Used Semicarbazide Linker.

Table II: Biological Activity of mPEG5000-EPOs Comparing Different Carbohydrate Modifying mPEG-Linker's

| mPEG-Linker | | Max. Hematocrit (%) * | Duration of Activity Relative to EPO (Days)** |
|---|---|---|---|
| $CH_3-(OCH_2CH_2)_n-O-CH_2-CO-NHNH_2$ | (Hydrazide) | 54 | 8 |
| $CH_3-(OCH_2CH_2)_n-NH-CO-NHNH_2$ | (Semicarbazide) | 61 | 20 |
| $CH_3-(OCH_2CH_2)_n-NH-CS-NHNH_2$ | (Thiosemicarbazide) | 59 | 14 |
| $CH_3-(OCH_2CH_2)_n-NH-CO-NHNH-CO-NHNH_2$ | (Carbonic Acid Dihydrazide) | 57 | 12 |
| $CH_3-(OCH_2CH_2)_n-O-CO-NHNH_2$ | (Hydrazide Carboxylate, 22 mPEGs) | 60 | |
| $CH_3-(OCH_2CH_2)_n-NH-CO-C_6H_4-NHNH_2$ | (Arylhydrazide) | 52 | 7 |

* Biological Assay Described in Experimental Section

** Days from EPO Maximum Hematocrit Level (46% on Day 4) Required to Reach EPO's Maximum Hematocrit Level After Attaining its Own Maximum Hematocrit Level

The hematocrit levels for EPO and mPEG5000-EPOs in mice are presented in Fig. 2. The 12PEG-EPO was made by coupling mPEG5000-hydrazide and reflects the maximum incorporation which could be achieved by this mPEG derivative under the experimental conditions given above. The 18PEG and 28PEG EPOs were made by coupling with mPEG5000-semicarbazide. The semicarbazide derivatives of mPEG result in much better biological activity than the hydrazide derivatives of mPEG due to the larger amounts of mPEG which can be incorporated using this mPEG derivative. All three mPEG5000-EPOs show increased maximum and prolonged activity when compared to native EPO. Thus modification of a protein's carbohydrate groups with PEG can yield a much more potent therapeutic protein.

For additional data on the effects of various mPEG hydrazide-modified EPOs employed in the experiments on hematocrit levels, see Figures 8-15. The mPEG modified EPO employed in the experiments depicted in Figures 8-15 were prepared using the appropriate water-soluble polymer reagent essentially as described for the other mPEG modified EPO molecules used the experiments depicted in Figures 2-5.

The in vivo biological activities (hematocrit levels) of the mPEG-EPOs linked via oxime-forming derivatives of mPEG is presented in Figures 16 and 17. To summarize, Figure 16 compares mPEG coupling using mPEG-O-CH2CH2-NH-CO-ONH2 ("A") and mPEG-O-CH2CH2-NH-CO-CH2-ONH2 ("C") linkers. Higher hematocrit levels of mPEG-EPO could be obtained using the "A" linker (corresponding to Formula XXX herein) having 18 mPEG molecules per molecule of EPO as compared to the "C" linker (corresponding to Formula XXXIII herein) having 31 mPEG molecules per molecule of EPO. Also notable was the higher hematocrit activity of the "C" linker (Formula XXXIII herein) having 25 mPEG molecules per molecule of EPO as compared to the same linker having 31 molecules of mPEG per molecule of EPO.

Figure 17 compares mPEG coupling using mPEG-O- CH2CH2-ONH2 (formula XXIII) ("B") linker at 22, 17, and 12 mPEG molecules per molecule of EPO. Highest hematocrit levels are obtained at the lowest degree of pegylation, and hematocrit was decreased inversely proportional to degree of pegylation. In all mPEG linkers using mPEG-O-CH2CH2-ONH2 oxime derivative however, hematocrits were higher and of increased duration as compared to native EPO.

Especially noteworthy is the biological activity of the 12mPEG-EPO of formula XXIII. Hydrazide derivitized 12 mPEG-EPO produces neither the degree nor duration of hematocrit elevation as that of the oxylamine derivitized EPO.

Antibody binding to mPEG-EPO

The antigenicity of the mPEG-EPOs was determined by using the Clinigen™ erythropoietin (EPO) EIA test kit. Briefly, the assay consists of a micro titre plate coated with a monoclonal antibody to EPO. EPO or mPEG-EPO is allowed to interact with the coated plate. After washing the plate a labeled polyclonal antibody to EPO is incubated on the plate. After substrate development the plate is read.

The results of the ELISA assay for hydrazide derivatized EPO, are presented in Figure 3. The mPEG-EPOs are presented as the approximate number of mPEGs attached for a given molecular weight of mPEG. For example, 12PEG-5k means about 12 mPEG molecules of a molecular weight of about 5000 were coupled to each molecule of EPO. The data indicates that as the number of mPEGs coupled to EPO are increased, the antigenicity of the protein is decreased. Similarly as the molecular weight of mPEG is increased, the antigenicity, i.e. the binding of the antibody, of the modified EPO also is decreased. Reacting oxidized EPO with a hydrazide derivative of mPEG did not reach the high coupling levels seen with semicarbazide, thiosemicarbazide, and carbonic acid dihydrazide PEG derivatives and thus could not give the large decreases in immunogenicity as seen with these other mPEG derivatives. Decreasing the antigenicity of a protein correlates to a decrease in the immunogenicity of a protein as well. Thus mPEG-EPO coupled to the carbohydrate groups of EPO may reduce any potential immunogenicity related to the protein with those derivatives of mPEG able to be coupled at high levels being the most effective.

For additional ELISA data with mPEG hydrazide derivatives see Figure 6.

The results of the ELISA assay for oxime derivatized EPO are presented in Figure 18. The data indicates that as the number of mPEGs coupled to EPO are increased, the antigenicity of the protein is decreased. Reacting oxidized EPO with a linker that resulted in comparatively low coupling levels (18 PEG-A, 17 PEG-B, 12 PEG-B) did not give the large decreases in immunogencity as seen with the comparatively high coupling level formulations (22 PEG-B, 25 PEG-C, and 31 PEG-C. Note that these differences in a linker's ability to decrease immunogenicity appear to be determined largely based on the coupling level (e.g. compare 12 PEG-B and 22PEG-B). Thus, mPEG coupled to carbohydrate groups of EPO through oxime linkages may reduce potential immunogencity related to the protein, with those derivatives of mPEG able to be coupled at high levels being the most effective.

Modification of Horseradish Peroxidase: Comparison of Hydrazide Versus Semicarbazide Coupling

Horseradish peroxidase (HRP) is a glycoprotein enzyme (oxido-reductase). HRP was modified with either mPEG5000-hydrazide or mPEG5000-semicarbazide in order to see whether another glycoprotein besides EPO could show the difference in modification between the two different carbohydrate modification reagents. In a typical experiment, horseradish peroxidase (2 mg) was placed in 100 mM sodium acetate, pH 5.6, total volume 0.8 ml. Enough 10 mg/ml solution of sodium periodate was added to give a final concentration of sodium periodate at 10 mMol. The oxidation went for 15 min at 0°C in the dark, after which time 0.33 ml 80 mMol Na2SO3 was added. After 5 minutes, the solution was concentrated and washed three times with 100 mMol sodium acetate, pH 4.2, in a micro concentrator. The oxidized horseradish peroxidase solution was then split in half, with one half receiving mg PEG5000-hydrazide and the other half receiving 30 mg mPEG5000-semicarbazide. The two oxidized horseradish peroxidase solutions were then stirred overnight at room temperature. The extent of PEG modification was determined by HPLC gel filtration using a Zorbax™ GF-250 column using a 0.1 M phosphate buffer, pH 7. The mPEG5000-hydrazide modified horseradish peroxidase had approximately 7 PEG molecules/HRP; the mPEG5000-semicarbazide modified horseradish peroxidase had approximately 19 PEG molecules/HRP. Thus modification of horseradish peroxidase with PEG being attached to its carbohydrate groups is more effective using a semicarbazide derivative of PEG than a hydrazide under the same experimental conditions.

Half-Life Determinations

Half-Life experiments were done in male Sprague-Dawley rats weighing about 0.3 kg. Three rats were used for each compound. The experimental details are as follows. Each rat was injected IV (intravenously) with 1 μg EPO or mPEG-EPO. For the hydrazide derivatized mPEG-EPO, the mPEG-EPO used was mPEG500 semicarbazide-18, prepared essentially as described in the section above on EPO modification with semicarbazides. Blood was withdrawn from each rat at 2, 5, 15, 45, 90 minutes and 3, 6, 24, 48, 54 hour time points. The blood was collected in heparinized tubes, and the plasma was isolated. The isolated plasma was tested

for EPO biological activity in an EPO dependent cell proliferation assay. The in vitro assay employed used an FDC-P1/ER cell line. This murine cell line incorporates the EPO receptor and is dependent on EPO for growth. The assay was performed as follows. The cells were grown (106/ml) in the absence of EPO for 24 hr after which time either EPO or mPEG-EPO at different concentrations is added to the cells. The cells were incubated for 42 hr, and then tritiated thymidine was added to the cells. After 6 hr the cells were harvested and counted. Cell growth was determined by the increased up-take of thymidine. Results are given in figure 7.

The EPO dependent cell proliferation assay was performed as described above using oxime-derivatized mPEG-EPO. Results are given in Figure 19.

In Vivo Assay: Anemic Mouse Model

In this assay mice are rendered anemic by injections for five consecutive days with TNF-alpha. To overcome the anemia the mice were injected SC (subcutaneously) with either EPO or mPEG-EPO (at 0.03 μg/dose) over the same five days or on just two of the five days that the mice receive TNF-alpha. Results are given in figure 15.

Equivalents

All publications and patents mentioned in the above specification are herein incorporated by reference. The foregoing written specification is considered to be sufficient to enable one skilled in the art to practice the invention. Indeed, various modifications of the above-described modes for carrying out the invention which are obvious to those skilled in the field of molecular biology or related fields are intended to be within the scope of the following claims.

**Claims**

**1.** A compound having the formula:

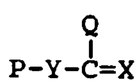

wherein X is O or S; Q is selected from the group consisting of $-NHNH_2$, and $-C_6H_4-NHNH_2$; and Y is selected from the group consisting of -O-, $-OCH_2-$, -NH-, -NHNH-, $-O-CO-CH_2CH_2-$ and -NHCO-NHNH-; and P is a water-soluble polymer.

**2.** A compound according to claim 1, said compound belonging to the group consisting of,
a compound having the formula:
(I) $P-O-CH_2-CO-NHNH_2$,
a compound having the formula:
(II) $P-O-CO-NHNH_2$,
a compound having the formula:
(III) $P-NH-CO-NHNH_2$,
a compound having the formula:
(IV) $P-NH-CS-NHNH_2$,
a compound having the formula:
(V) $P-NHCO-NHNHCO-NHNH_2$,
a compound having the formula:
(VI) $P-NHNHCONHNH_2$,
a compound having the formula:
(VII) $P-NHNHCSNHNH_2$,
a compound having the formula:
(VIII) $P-NH-CO-C_6H_4-NHNH_2$, and
a compound having the formula:
(IX) $P-O-CO-CH_2CH_2-CO-NHNH_2$.

**3.** A compound having the formula:

P-Y-X-Q

wherein X is C=O, C=S, $CH_2$ or CHOH; Q is selected from the group consisting of $-ONH_2$ and $-CH_2-ONH_2$ and Y is selected from the group consisting of $-O-CH_2CH_2-$, $-O-CH_2CH_2-O-$, $-O-CH_2CH_2-N-$, $-O-CH_2CH_2-S-$, and $-O-CH_2CH_2-NH-$; and P is a water soluble polymer.

**4.** A compound according to claim 3, said compound belonging to the group consisting of,

a compound having the formula:

(XIX) $P-O-CH_2CH_2-CO-ONH_2$,

a compound having the formula:

(XX) $P-O-CH_2CH_2-O-CO-ONH_2$,

a compound having the formula:

(XXI) $P-O-CH_2CH_2-NH-CO-ONH_2$,

a compound having the formula:

(XXII) $P-O-CH_2CH_2-NH-CS-ONH_2$,

a compound having the formula:

(XXIII) $P-O-CH_2CH_2-ONH_2$,

a compound having the formula:

(XXIV) $P-O-CH_2CH_2-NH-CO-CH_2ONH_2$,

a compound having the formula:

(XXV) $P-O-CH_2CH_2-O-CO-CH_2-ONH_2$,

a compound having the formula:

(XXVI) $P-O-CH_2CH_2-CH(OH)-CH_2-ONH_2$,

a compound having the formula:

(XXVII) $P-O-CH_2CH_2-CO-CH_2-ONH_2$.

**5.** A compound belonging to the group consisting of

a compound having the formula:

(X) $[P-O-CH_2-CO-NHN=CH-]_n-Z$,

a compound having the formula:

(XI) $[P-O-CO-NHN=CH-]_n-Z$,

a compound having the formula:

(XII) $[P-CH-CO-NHN=CH-]_n-Z$,

a compound having the formula:

(XIII) $[P-NH-CS-NHN=CH-]_n-Z$,

a compound having the formula:

(XIV) $[P-NHCO-NH-NHNHCO-NHN=CH-]_n-Z$,

a compound having the formula:

(XV) $[P-NHNCON=CH-]_n-Z$,

a compound having the formula:

(XVI) $[P-NHNCSN=CH-]_n-Z$,

a compound having the formula:

(XVII) $[P-NH-CO-C_6H_4-NHN=CH-]_n-Z$; and

a compound having the formula:

(XVIII) $[P-O-CO-CH_2CH_2-CO-NHN=CH-]_n-Z$

wherein Z is a polypeptide, n is 1 to x, x being the number of oxidation activatable groups on Z, and P is a water-soluble polymer.

**6.** A compound belonging to the group consisting of,

a compound having the formula:

(XXVIII) $[P-O-CH_2CH_2-CO-ON=CH-]_n-Z$;

a compound having the formula:

(XXIX) $[P-O-CH_2CH_2-O-CO-ON=CH-]_n-Z$;

a compound having the formula:

(XXX) $[P-O-CH_2CH_2-NH-CO-ON=CH-]_n-Z$;

a compound having the formula:

(XXXI) $[P-O-CH_2CH_2-NH-CS-ON=CH-]_n-Z$;

a compound having the formula:

(XXXII) $[P-O-CH_2CH_2-ON=CH-]_n-Z$;

a compound having the formula:

(XXIII) $[P-O-CH_2CH_2-NH-CO-CH_2-ON=CH-]_n-Z$;

a compound having the formula:

(XXXIV) $[P-O-CH_2CH_2-O-CO-CH_2-ON=CH-]_n-Z$;

a compound having the formula:

(XXXV) $[P-O-CH_2CH_2-CH(OH)-CH_2-ON=CH-]_n-Z$; and

a compound having the formula:

(XXXVI) $[P-O-CH_2CH_2-CO-CH_2-ON=CH-]_n-Z$,

wherein Z is a polypeptide, n is 1 to x, x being the number of oxidation activatable groups on Z, and P is a water-soluble polymer.

**7.** A compound according to claim 5 or claim 6, wherein n is 10-36.

**8.** A compound according to claim 7 wherein n is 17-25.

**9.** A compound according to any of claims 5 to 8, wherein Z is selected from the group consisting of hormones, lymphokines, cytokines, growth factors, enzymes, vaccine antigens, and antibodies.

**10.** A compound according to claim 9, wherein Z is erythropoietin.

**11.** A compound according to any preceding claim, wherein P is selected from the group consisting of polyethylene glycol homopolymers, polypropylene glycol homopolymers, copolymers of ethylene glycol with propylene glycol, wherein said homopolymers and copolymers are unsubstituted or substituted at one end with an alkyl group, polyoxyethylated polyols, polyvinyl alcohol, polysaccharides, polyvinyl ethyl ethers, $\alpha,\beta$-Poly[(2-hydroxyethyl)-DL-aspartamide], RO-PEG, where R may be alkyl, aryl, alkylaryl, aroyl, alkanoyl, benzoyl, arylalkylethers, cycloalkyl, cycloalkylaryl, and derivatives of said polymers.

**12.** A compound according to claim 11, wherein P is monomethoxypoly(ethylene glycol) having an average molecular weight in the range of 2000-12000, more preferably 5000.

**13.** A water-soluble polymer modified polypeptide, said modified polypeptide produced by a method comprising the step,

mixing a compound according to any of claims 1 to 4 with a polypeptide for modification.

**14.** A modified polypeptide according to claim 13, wherein said polypeptide for modification is selected from the group consisting of hormones, lymphokines, cytokines, growth factors, enzymes, vaccine antigens, and antibodies.

**15.** A modified polypeptide according to claim 13, wherein said polypeptide for modification is an antibody, said method further comprising the step of combining said antibody with a compound capable of specifically binding to a binding site on said antibody, prior to said mixing step.

**16.** A modified polypeptide according to claim 13, wherein said polypeptide for modification is an enzyme, said method further comprising the step of combining said polypeptide with a substrate for said enzyme, prior to said mixing step.

**17.** A modified polypeptide according to claim 13, wherein said polypeptide is erythropoietin.

**18.** A composition comprising a polypeptide according to any of claims 13 to 17 and a pharmaceutically acceptable carrier.

**19.** A method of activating polypeptides for conjugation with compounds selected from the group consisting of compounds II, III, IV, V, XIX, XXI, XXIII and XXIV said method comprising the step,

mixing a polypeptide for activation with an oxidizing agent.

**20.** A method according to claim 19, wherein said oxidizing agent is sodium periodate, at a concentration of 10-40 micromoles per milligram of protein, and said mixing step takes place at a temperature in the range of -10 to 50°C for a period of time between 1 minute and 3 days.

**21.** A kit for modifying polypeptides with water-soluble polymers, said kit comprising,

a water-soluble polymer according to claim 1 or 2, an oxidizing agent and a polypeptide for modification.

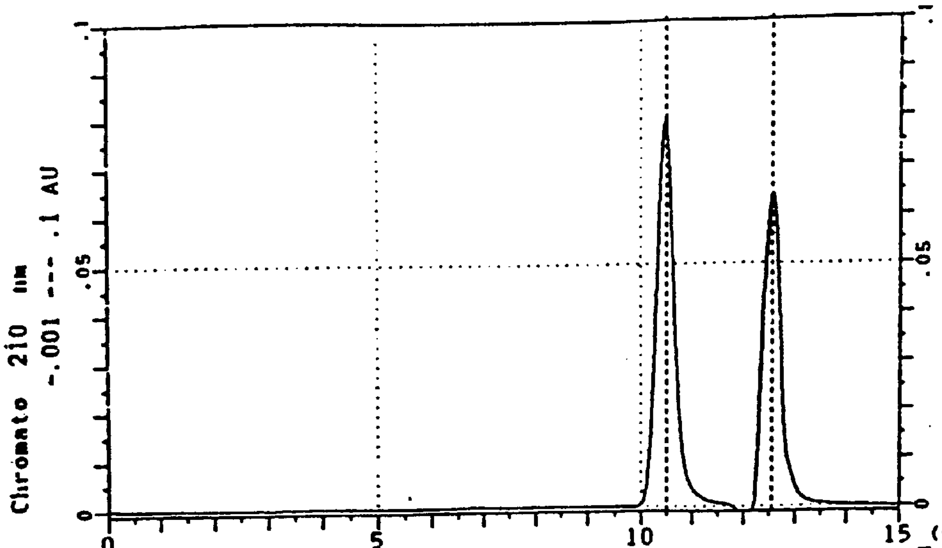


Fig. 1a
EPO

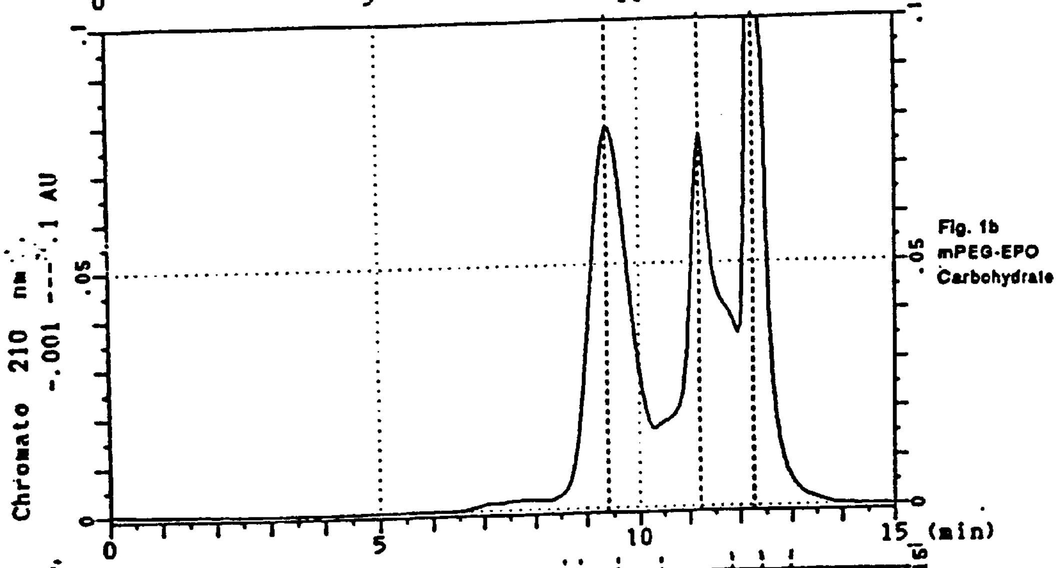


Fig. 1b
mPEG-EPO
Carbohydrate Modification

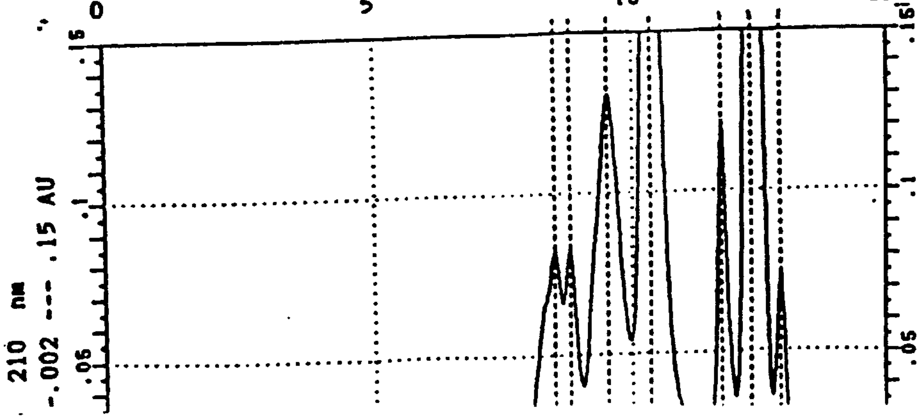


Fig. 1c
mPEG-EPO
Amino Acid Modification

# Figure 2: Hematocrit Levels in Mice

## EPO and mPEG5000-EPOs

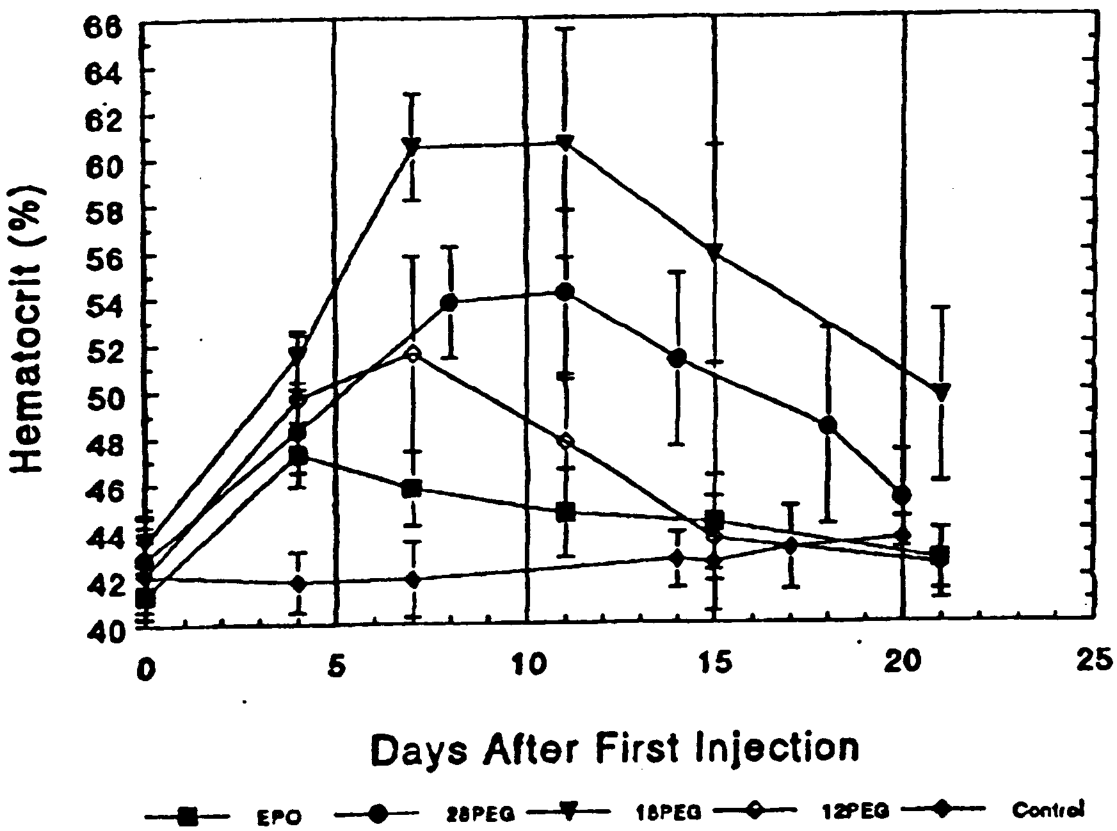

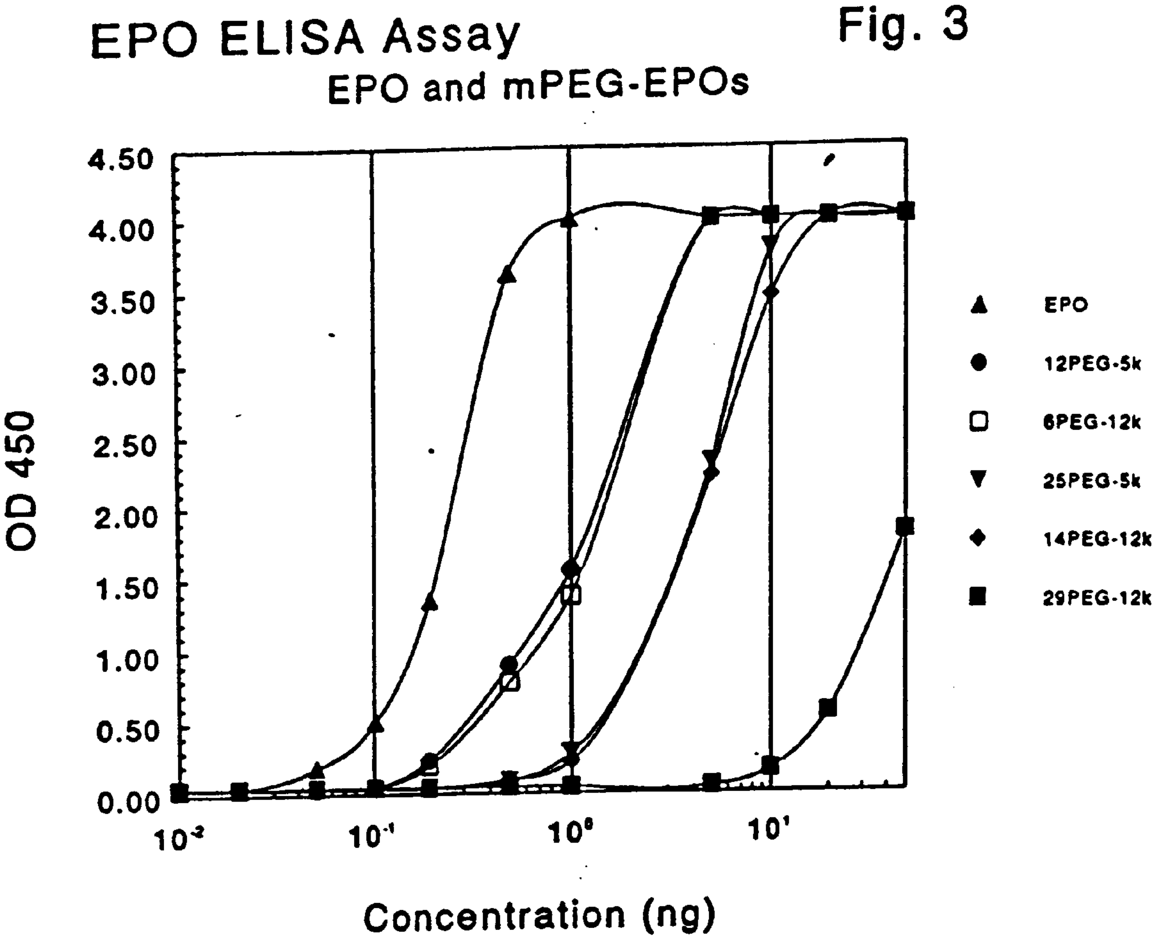
EPO ELISA Assay
EPO and mPEG-EPOs
OD 450
4.50
4.00
3.50
3.00
2.50
2.00
1.50
1.00
0.50
0.00
$10^{-2}$
$10^{-1}$
$10^{0}$
$10^{1}$
Concentration (ng)
EPO
12PEG-5k
6PEG-12k
25PEG-5k
14PEG-12k
29PEG-12k

Fig. 3

## Figure 4: Hematocrit Levels in Mice
### Hydrazide and Semicarbazide Comparison

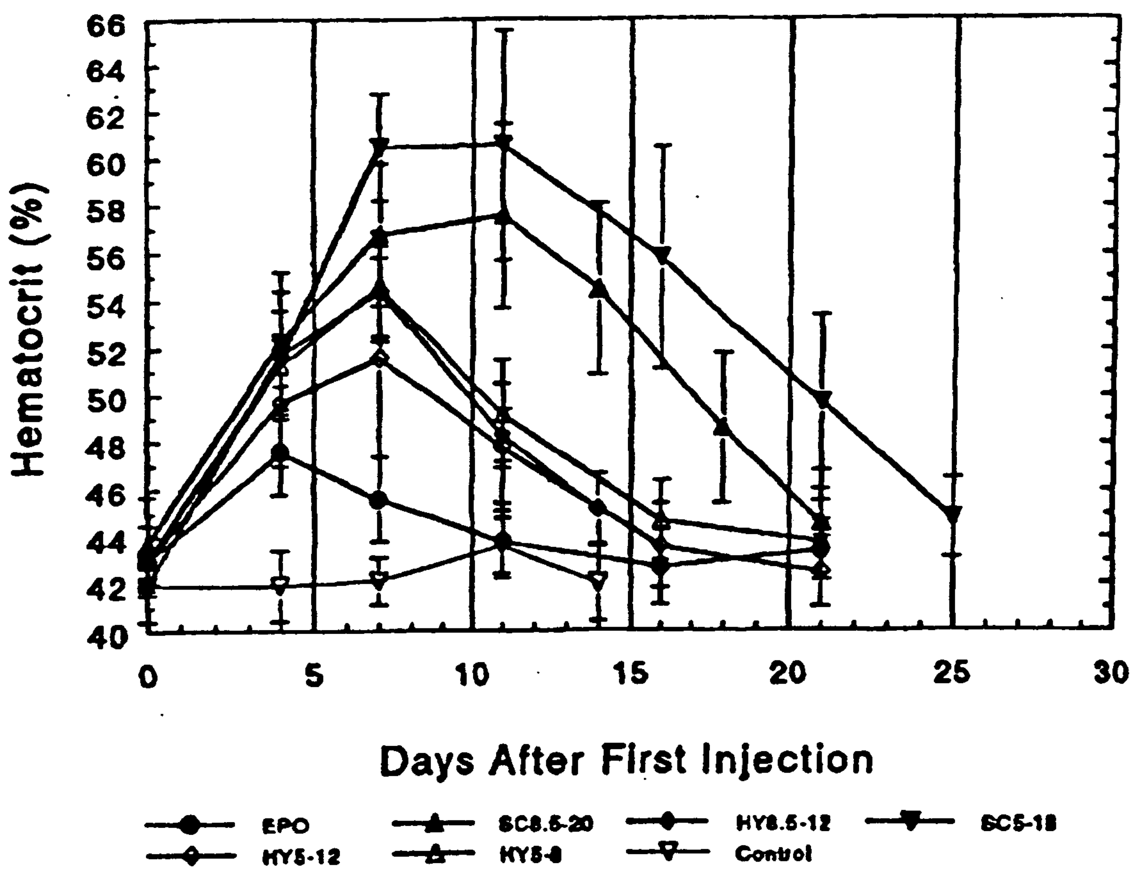

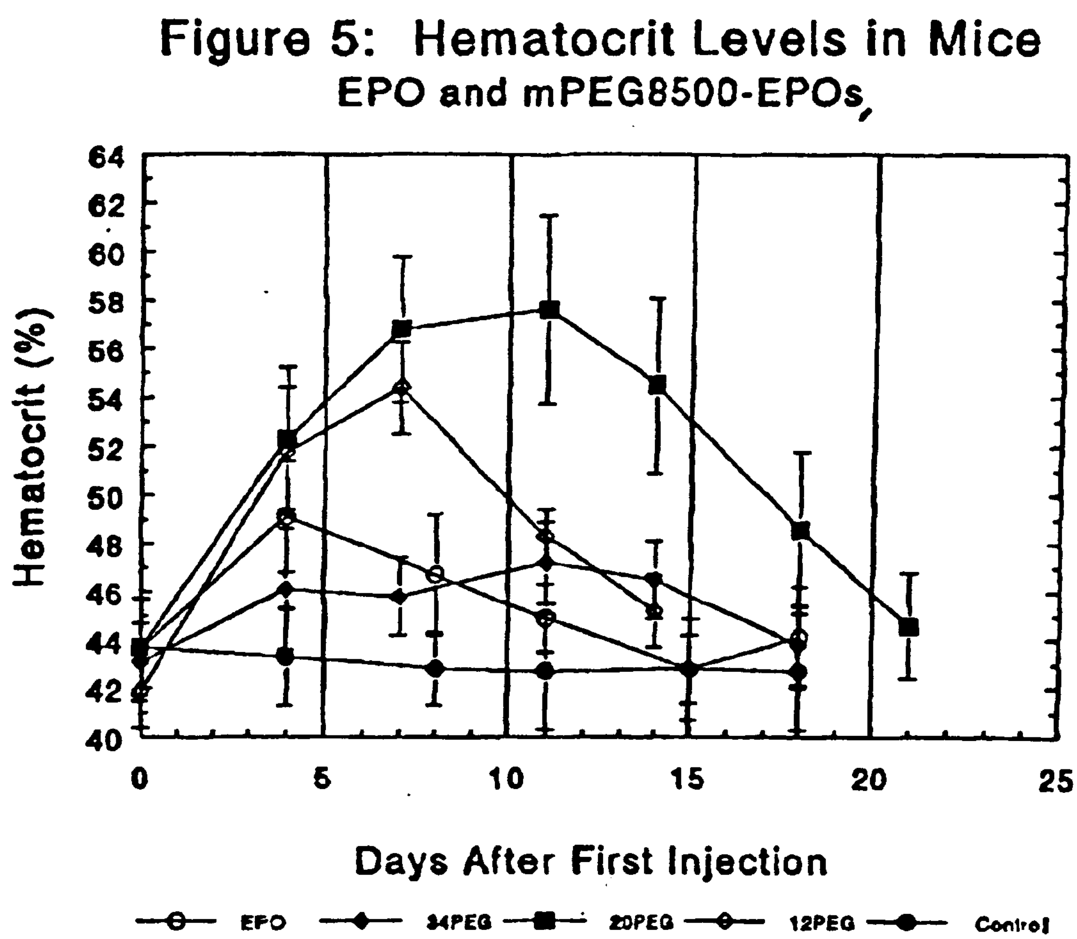
Figure 5: Hematocrit Levels in Mice
EPO and mPEG8500-EPOs
Hematocrit (%)
64
62
60
58
56
54
52
50
48
46
44
42
40
0
5
10
15
20
25
Days After First Injection
EPO
34PEG
20PEG
12PEG
Control

FIGURE 6

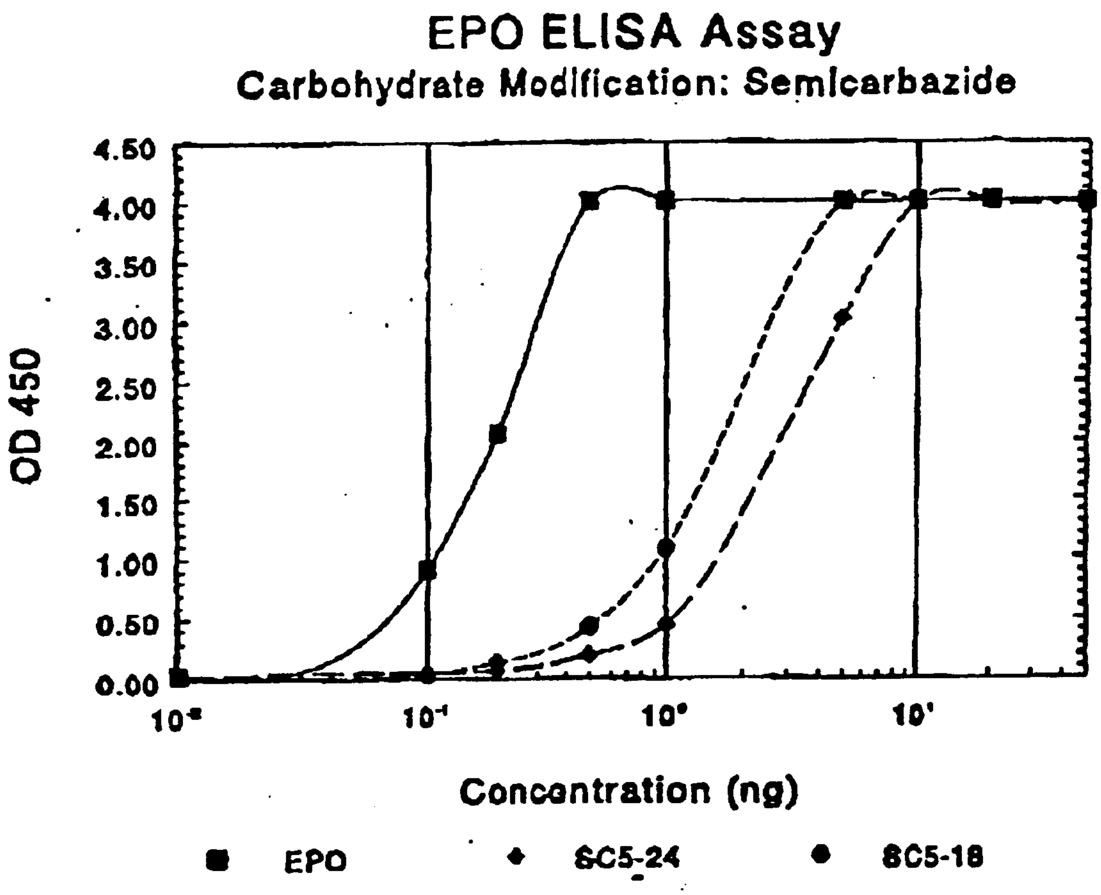
EPO ELISA Assay
Carbohydrate Modification: Semicarbazide
OD 450
4.50
4.00
3.50
3.00
2.50
2.00
1.50
1.00
0.50
0.00
10⁻¹
10⁰
10¹
Concentration (ng)
EPO
SC5-24
SC5-18

FIGURE 7

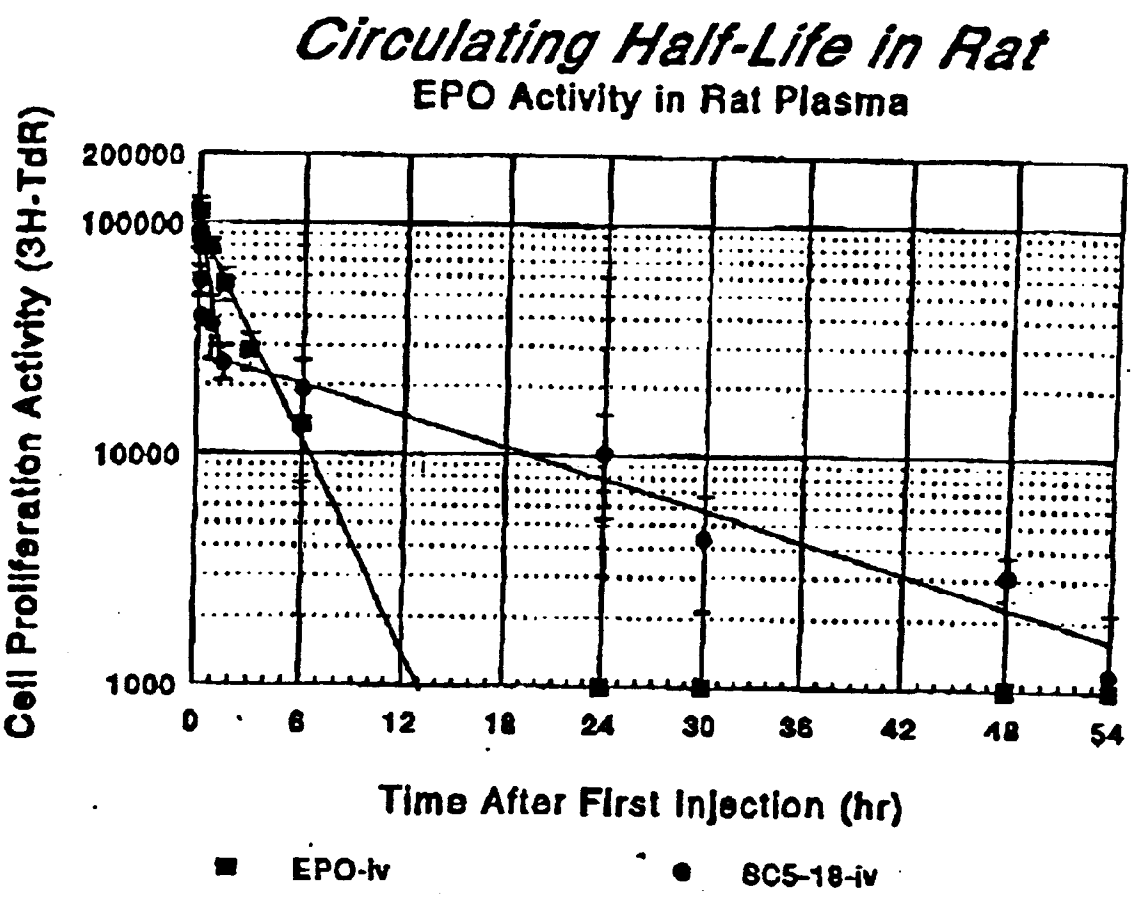
Circulating Half-Life in Rat
EPO Activity in Rat Plasma
Cell Proliferation Activity (3H-TdR)
200000
100000
10000
1000
0
6
12
18
24
30
36
42
48
54
Time After First Injection (hr)
EPO-iv
8C5-18-iv

FIGURE 8

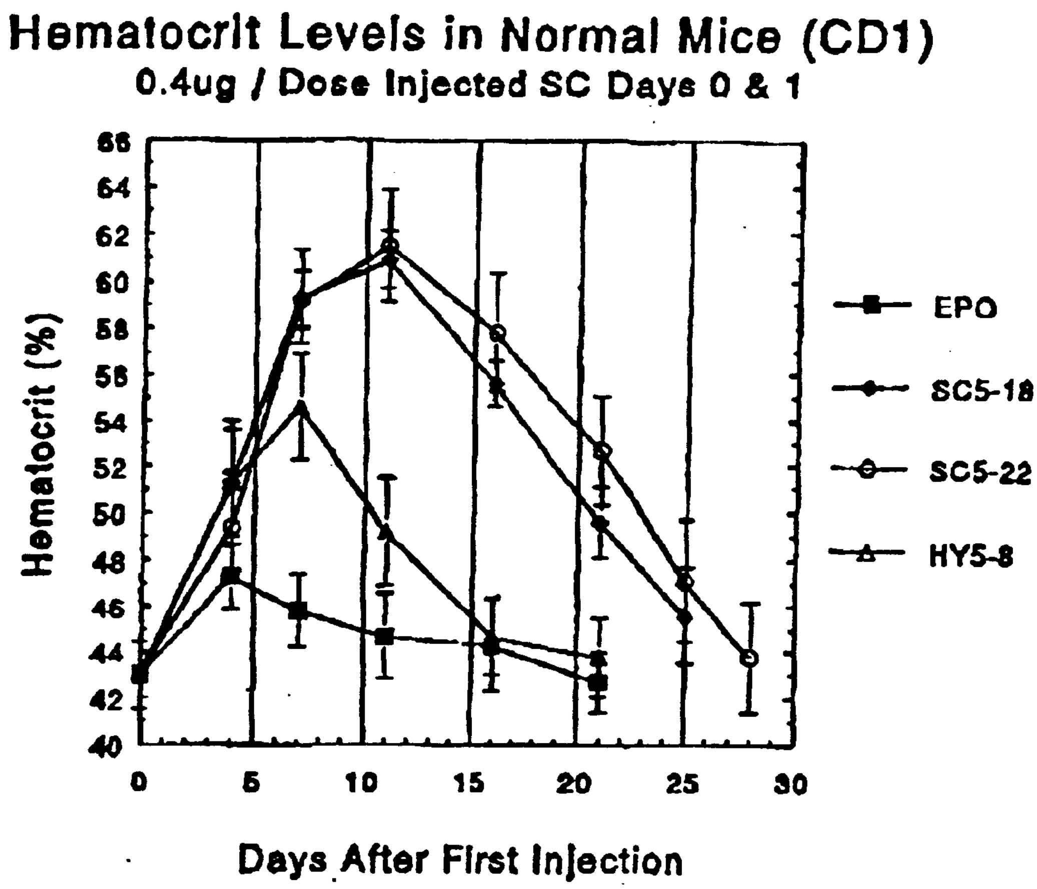
Hematocrit Levels in Normal Mice (CD1)
0.4ug / Dose Injected SC Days 0 & 1
Hematocrit (%)
66
64
62
60
58
56
54
52
50
48
46
44
42
40
0
5
10
15
20
25
30
Days After First Injection
EPO
SC5-18
SC5-22
HY5-8

FIGURE 9

## Hematocrit Levels in Normal Mice (CD1)

**0.4ug / Dose Injected SC Days 0 & 1**

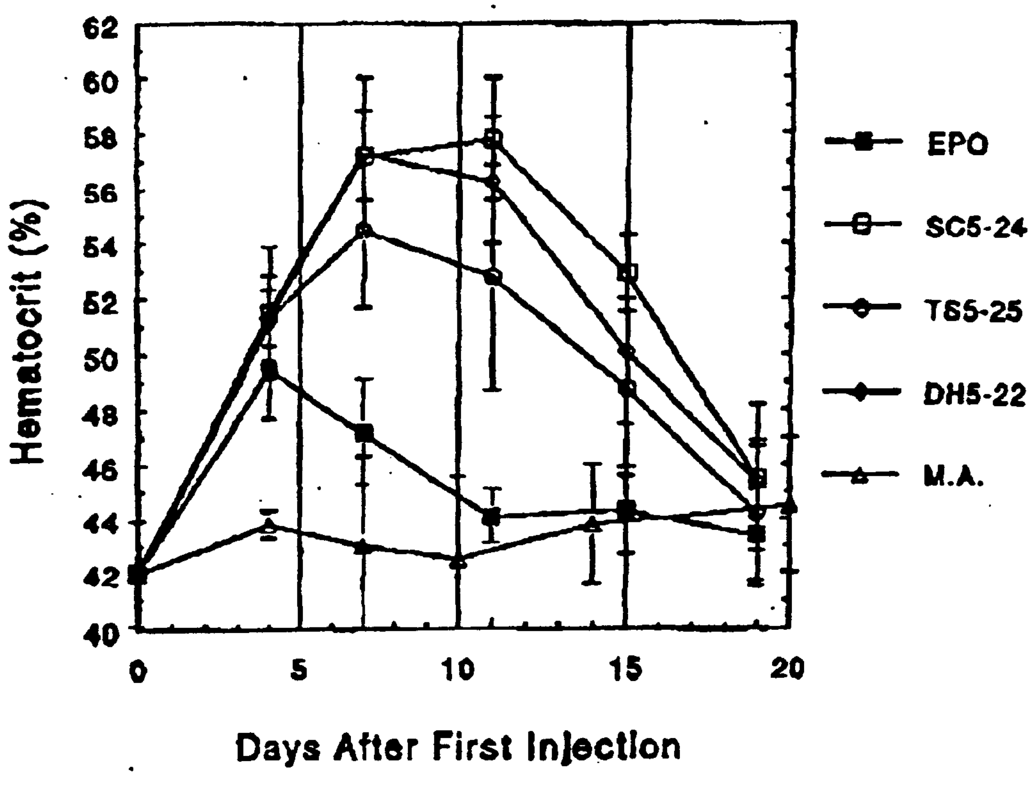

FIGURE 10

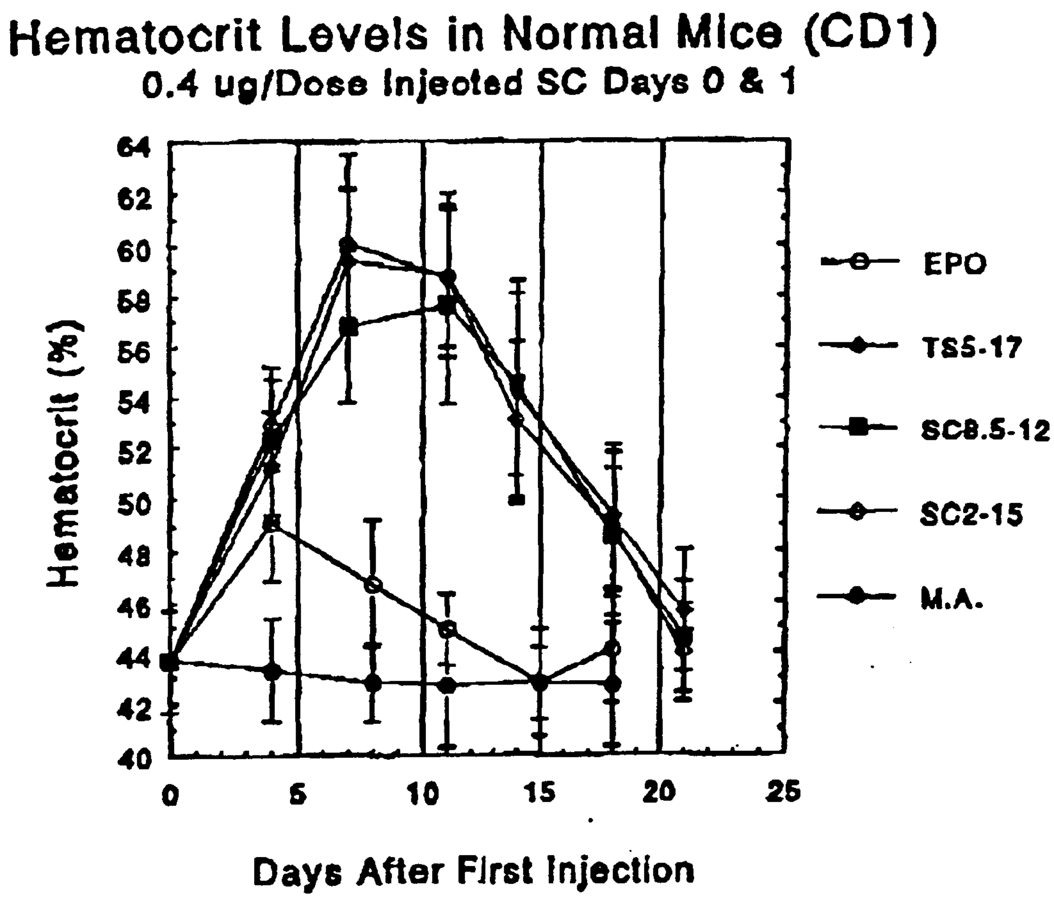
Hematocrit Levels in Normal Mice (CD1)
0.4 ug/Dose Injected SC Days 0 & 1
Hematocrit (%)
64
62
60
58
56
54
52
50
48
46
44
42
40
0
5
10
15
20
25
Days After First Injection
EPO
TS5-17
SC8.5-12
SC2-15
M.A.

FIGURE 11

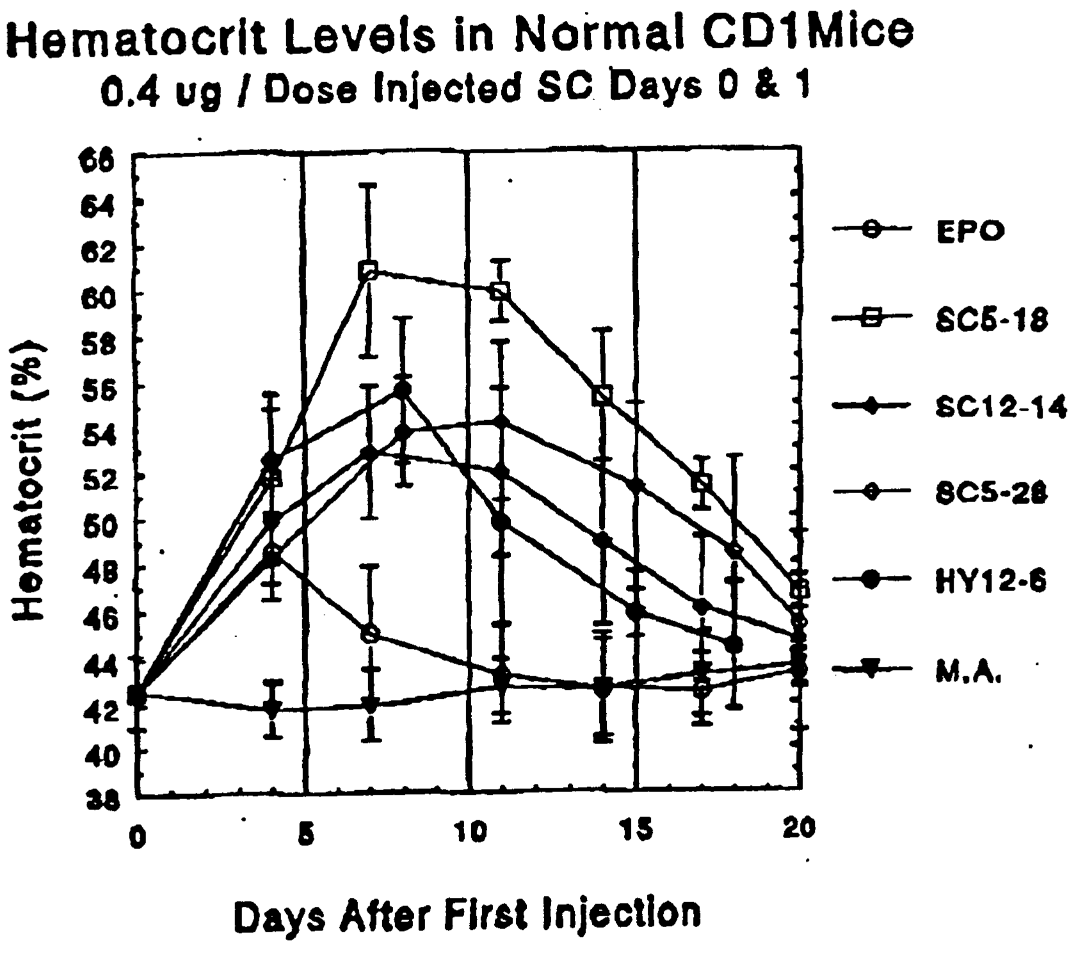
Hematocrit Levels in Normal CD1Mice
0.4 ug / Dose Injected SC Days 0 & 1
66
64
62
60
58
56
54
52
50
48
46
44
42
40
38
Hematocrit (%)
0
5
10
15
20
Days After First Injection
EPO
SC5-18
SC12-14
SC5-28
HY12-6
M.A.

FIGURE 12

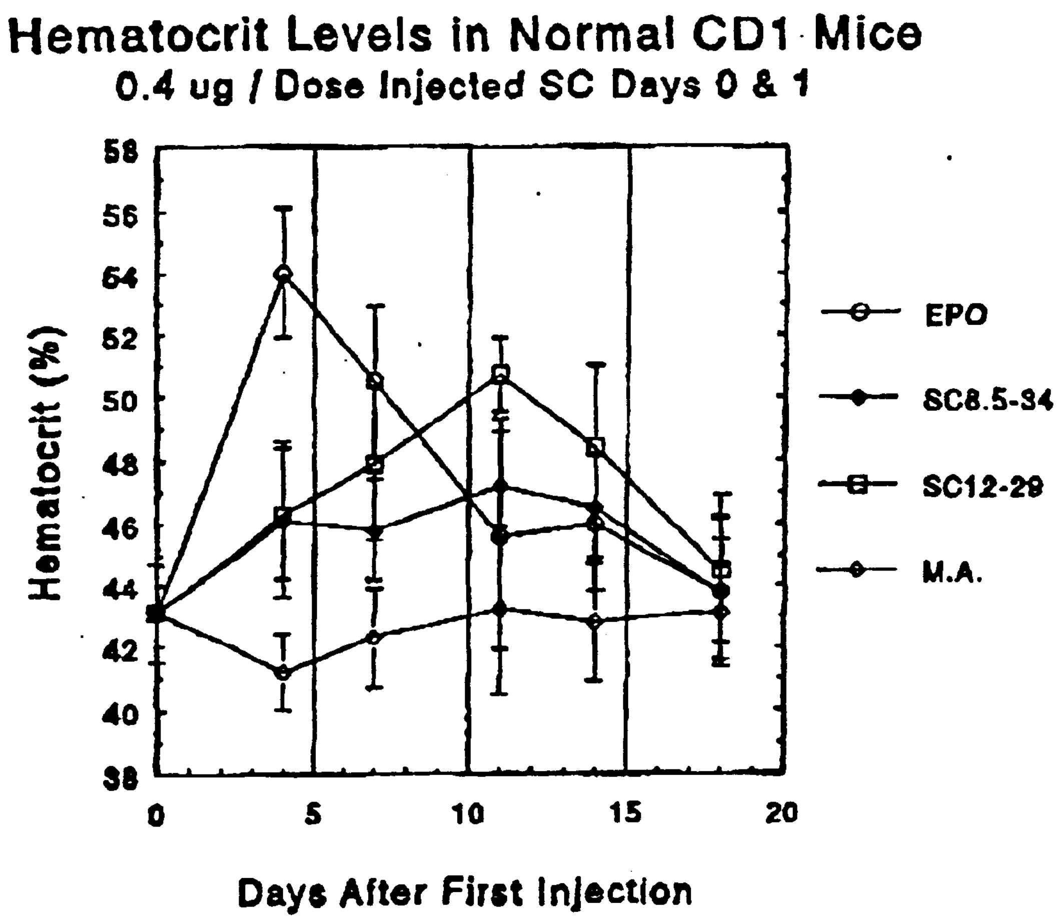
Hematocrit Levels in Normal CD1 Mice
0.4 ug / Dose Injected SC Days 0 & 1
Hematocrit (%)
58
56
54
52
50
48
46
44
42
40
38
0
5
10
15
20
Days After First Injection
EPO
SC8.5-34
SC12-29
M.A.

FIGURE 13

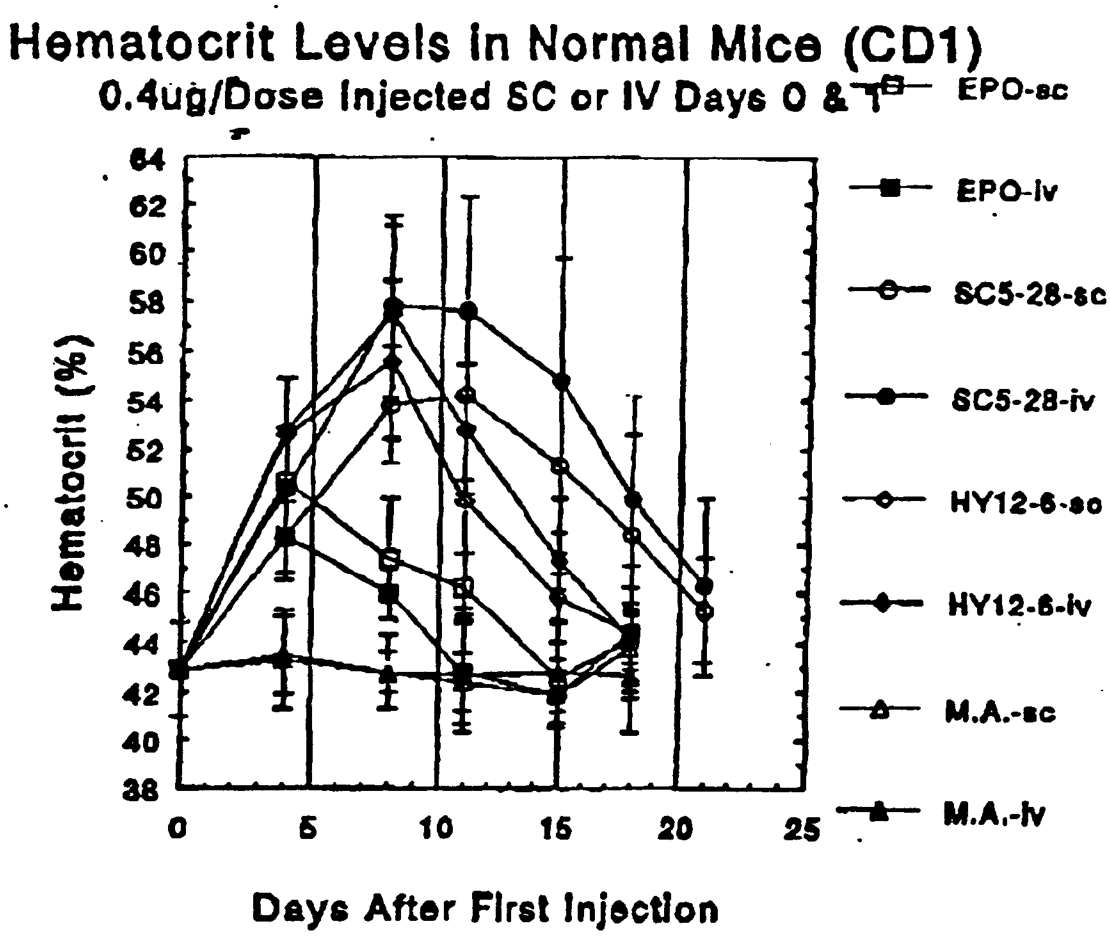
Hematocrit Levels in Normal Mice (CD1)
0.4ug/Dose Injected SC or IV Days 0 & 7
Hematocrit (%)
64
62
60
58
56
54
52
50
48
46
44
42
40
38
0
5
10
15
20
25
Days After First Injection
EPO-sc
EPO-iv
SC5-28-sc
SC5-28-iv
HY12-6-sc
HY12-6-iv
M.A.-sc
M.A.-iv

FIGURE 14

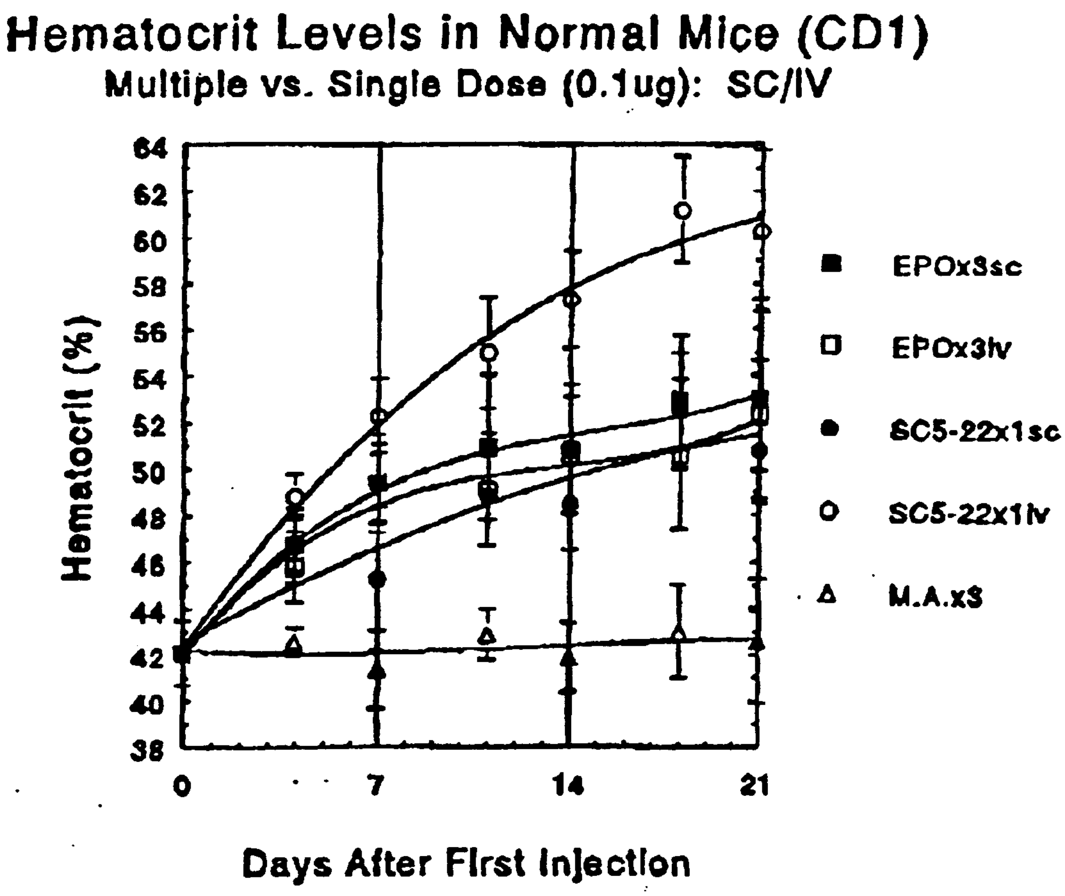
Hematocrit Levels in Normal Mice (CD1)
Multiple vs. Single Dose (0.1ug): SC/IV
Hematocrit (%)
64
62
60
58
56
54
52
50
48
46
44
42
40
38
0
7
14
21
Days After First Injection
EPOx3sc
EPOx3iv
SC5-22x1sc
SC5-22x1iv
M.A.x3

FIGURE 15

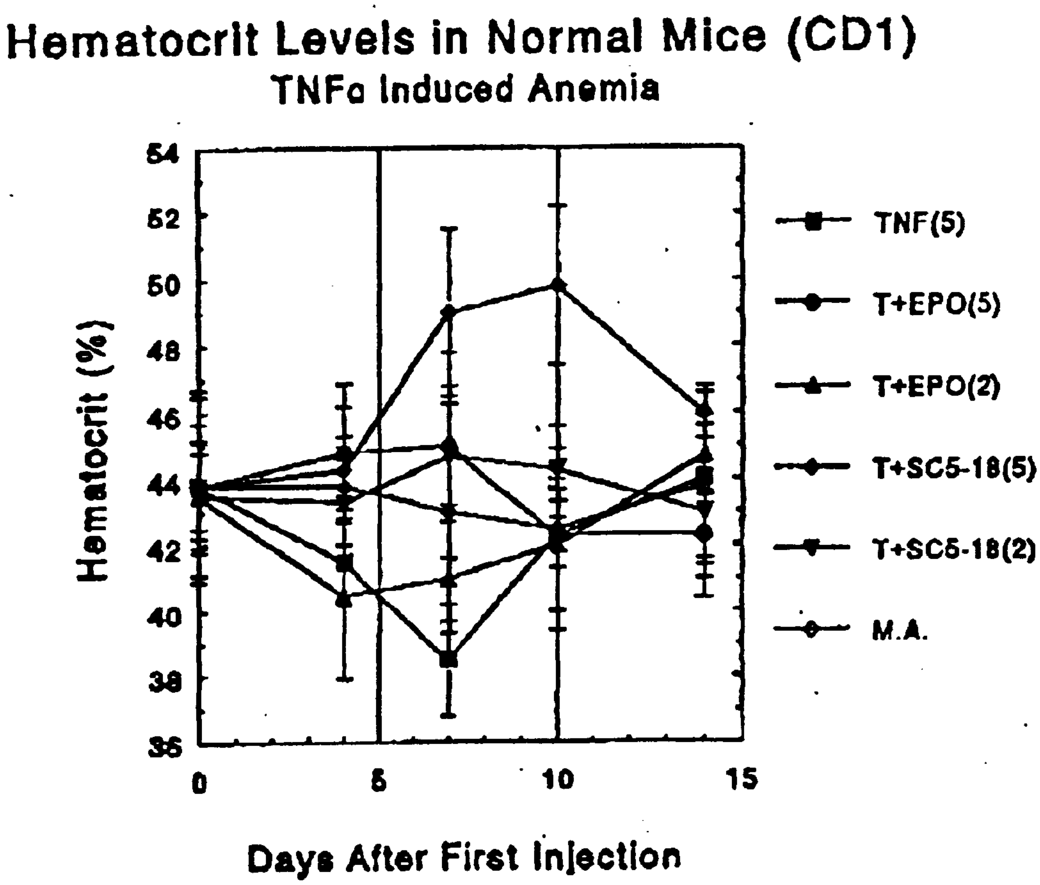
Hematocrit Levels in Normal Mice (CD1)
TNFα Induced Anemia
Hematocrit (%)
54
52
50
48
46
44
42
40
38
36
0
5
10
15
Days After First Injection
TNF(5)
T+EPO(5)
T+EPO(2)
T+SC5-18(5)
T+SC5-18(2)
M.A.

Figure 16

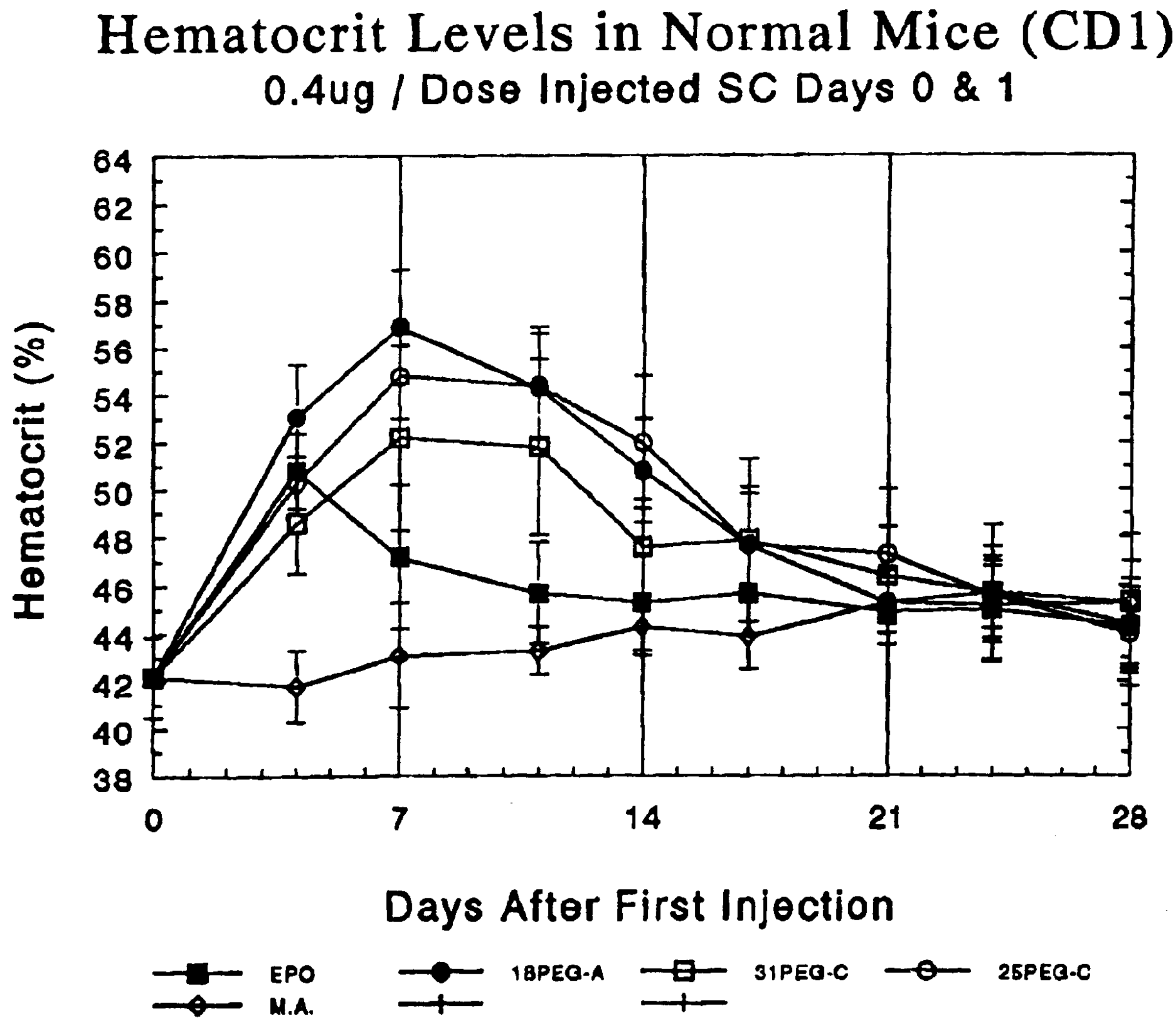
Hematocrit Levels in Normal Mice (CD1)
0.4ug / Dose Injected SC Days 0 & 1
Hematocrit (%)
64
62
60
58
56
54
52
50
48
46
44
42
40
38
0
7
14
21
28
Days After First Injection
EPO
18PEG-A
31PEG-C
25PEG-C
M.A.

Figure 17

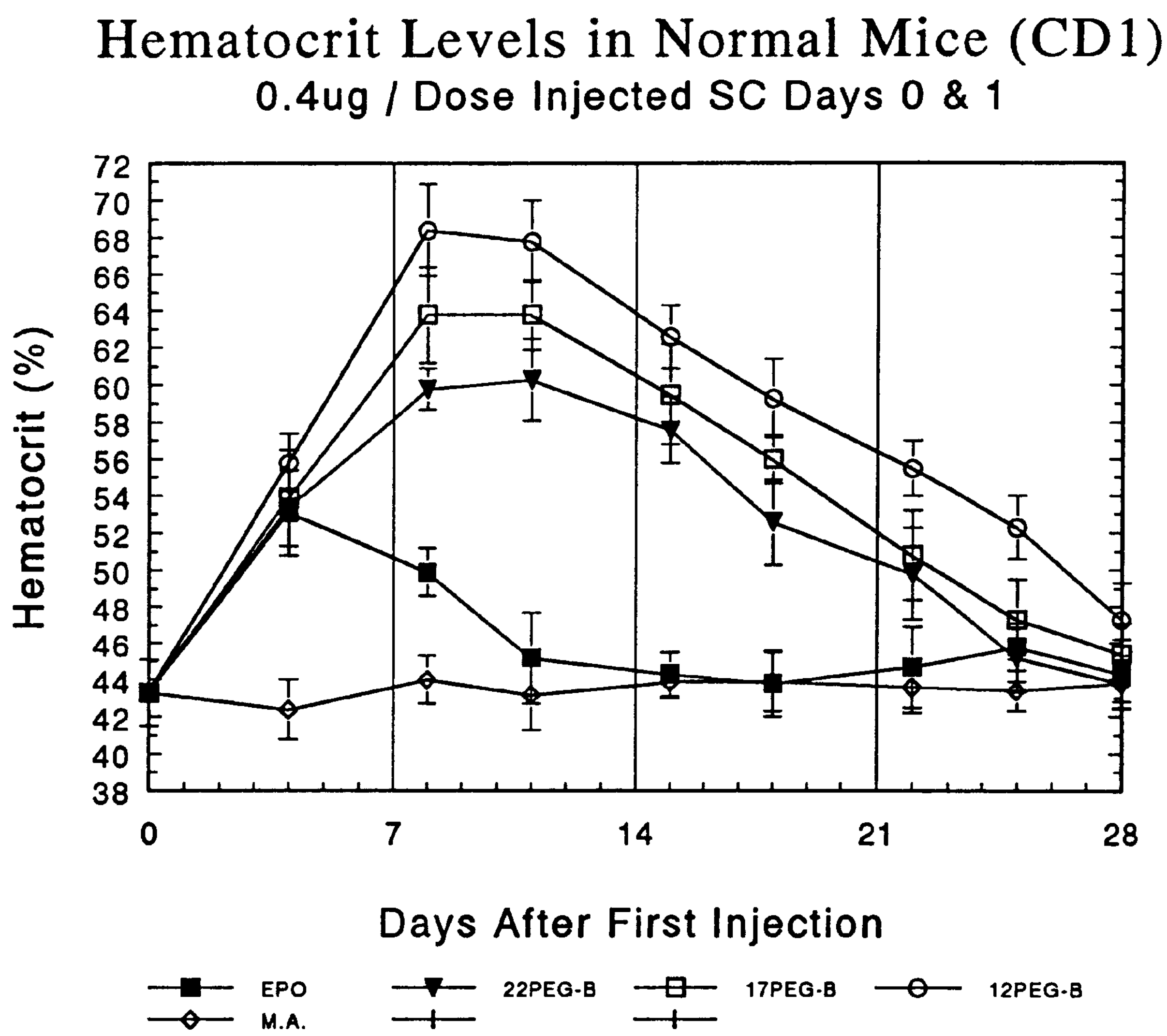
Hematocrit Levels in Normal Mice (CD1)
0.4ug / Dose Injected SC Days 0 & 1
Hematocrit (%)
72
70
68
66
64
62
60
58
56
54
52
50
48
46
44
42
40
38
0
7
14
21
28
Days After First Injection
EPO
22PEG-B
17PEG-B
12PEG-B
M.A.

Figure 18

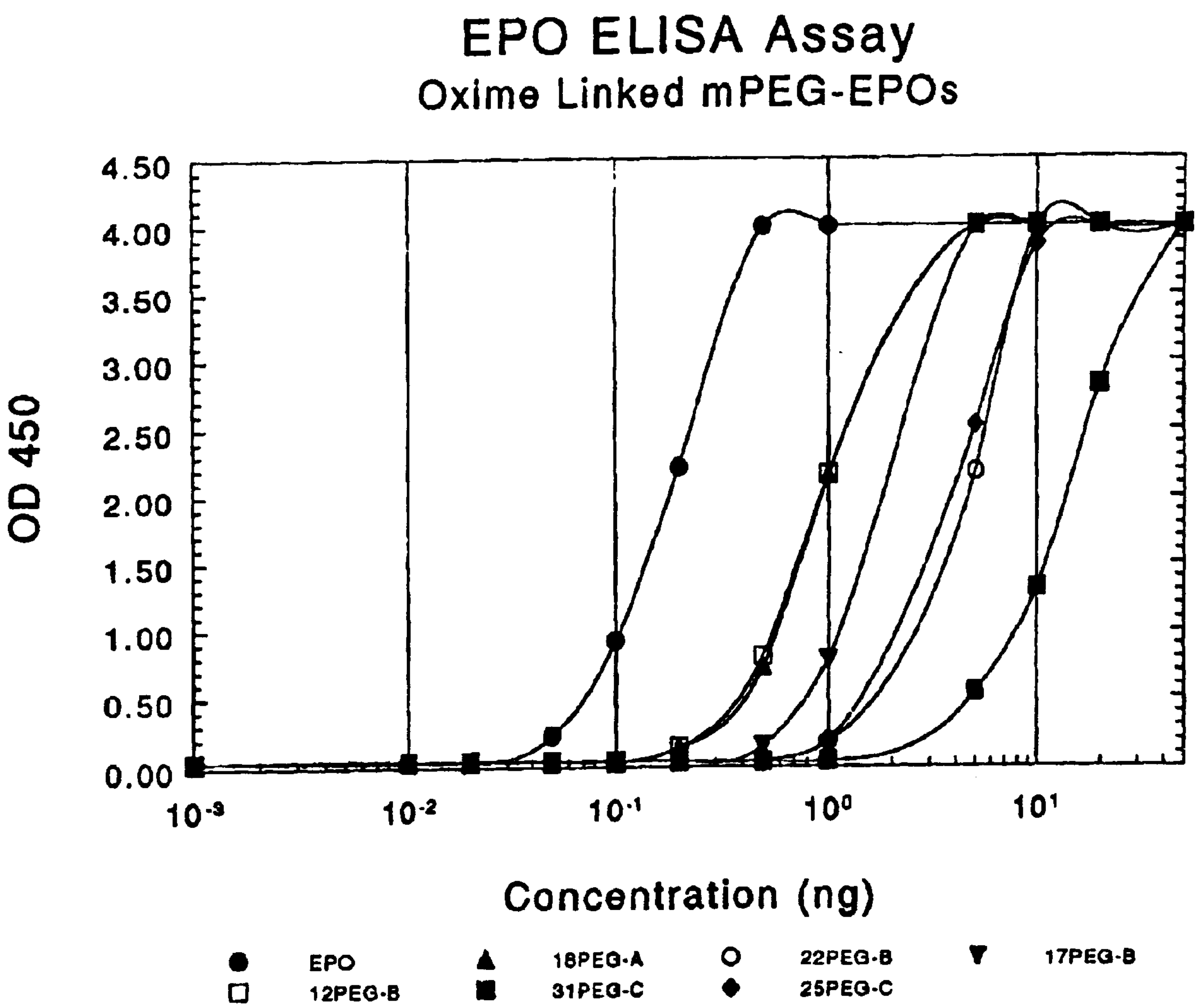
EPO ELISA Assay
Oxime Linked mPEG-EPOs
OD 450
4.50
4.00
3.50
3.00
2.50
2.00
1.50
1.00
0.50
0.00
$10^{-3}$
$10^{-2}$
$10^{-1}$
$10^{0}$
$10^{1}$
Concentration (ng)
EPO
18PEG-A
22PEG-B
17PEG-B
12PEG-B
31PEG-C
25PEG-C

Figure 19

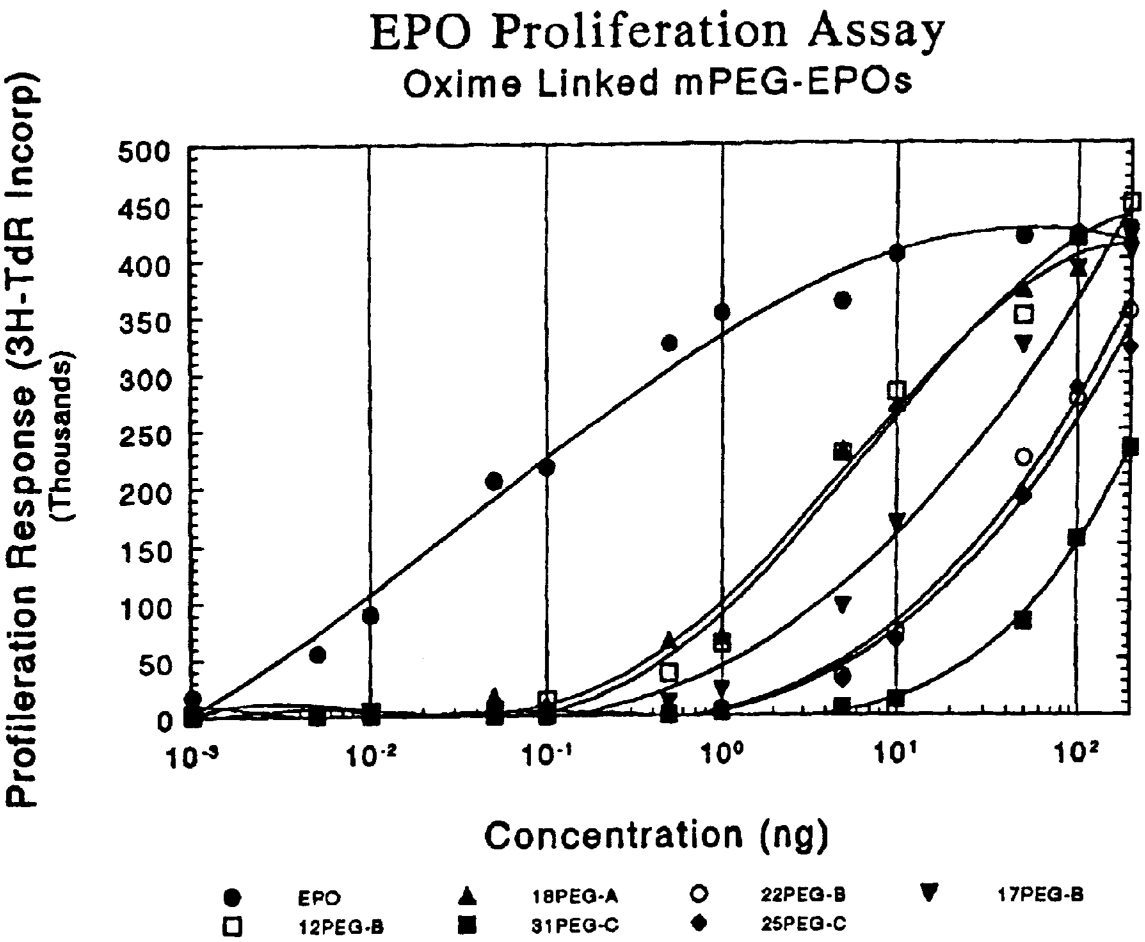
EPO Proliferation Assay
Oxime Linked mPEG-EPOs
Profileration Response (3H-TdR Incorp)
(Thousands)
500
450
400
350
300
250
200
150
100
50
0
$10^{-3}$
$10^{-2}$
$10^{-1}$
$10^{0}$
$10^{1}$
$10^{2}$
Concentration (ng)
EPO
18PEG-A
22PEG-B
17PEG-B
12PEG-B
31PEG-C
25PEG-C

Figure 20

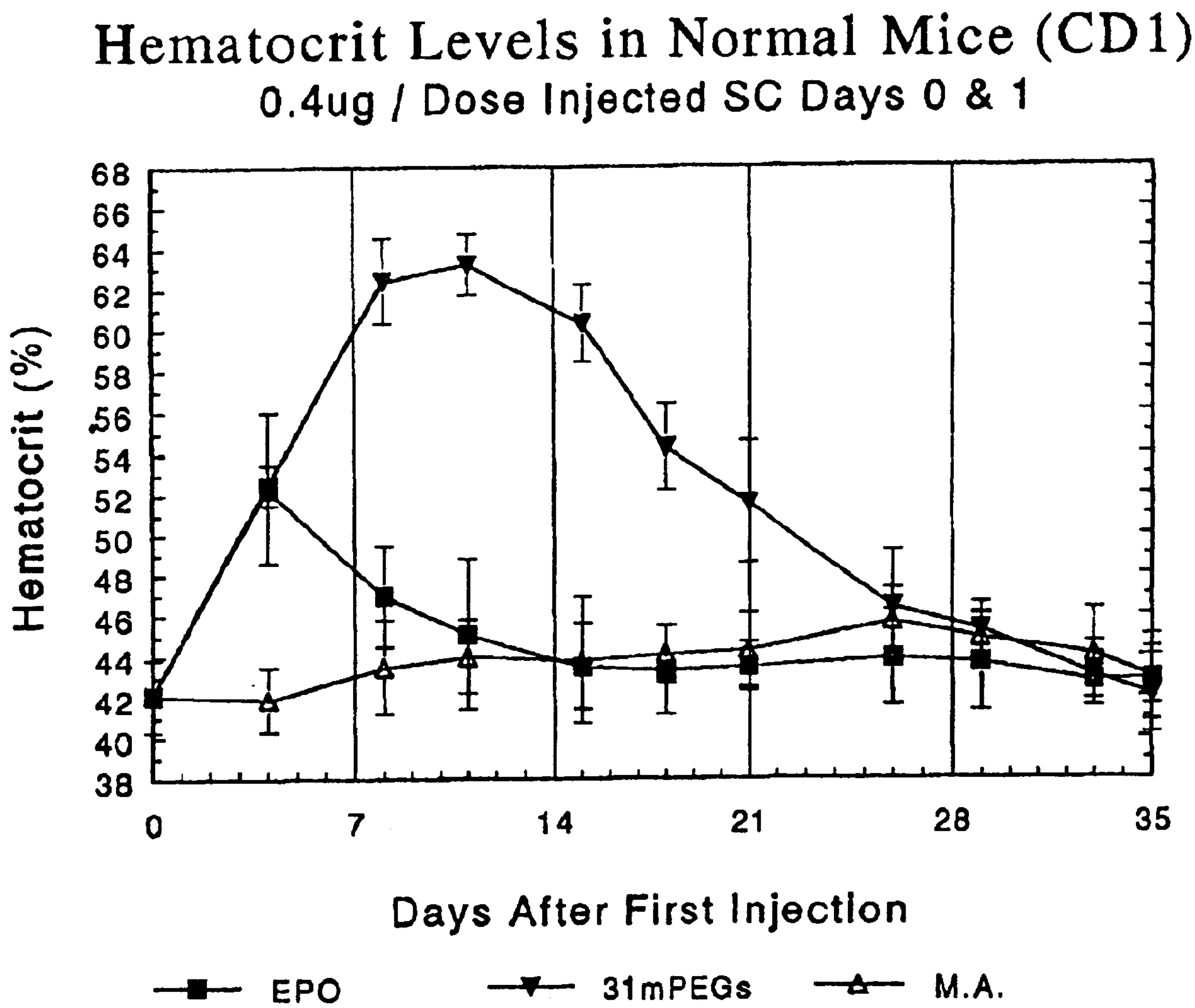
Hematocrit Levels in Normal Mice (CD1)
0.4ug / Dose Injected SC Days 0 & 1
Hematocrit (%)
68
66
64
62
60
58
56
54
52
50
48
46
44
42
40
38
0
7
14
21
28
35
Days After First Injection
EPO
31mPEGs
M.A.